(19) **Europäisches Patentamt**
**European Patent Office**
**Office européen des brevets**

(11) **EP 3 721 435 B1**

(12) **EUROPEAN PATENT SPECIFICATION**

(45) Date of publication and mention
of the grant of the patent:
**07.05.2025 Bulletin 2025/19**

(21) Application number: **18885752.8**

(22) Date of filing: **01.11.2018**

(51) International Patent Classification (IPC):
**G16H 20/10** (2018.01)     **A61B 5/00** (2006.01)
**G06Q 50/22** (2024.01)

(52) Cooperative Patent Classification (CPC):
**A61B 5/7275; A61B 5/0022; A61B 5/16;
A61B 5/4845; A61B 5/746; G06Q 50/22;
G16H 10/20; G16H 50/30**

(86) International application number:
**PCT/SE2018/051118**

(87) International publication number:
**WO 2019/112504 (13.06.2019 Gazette 2019/24)**

(54) **METHOD AND DEVICE FOR ESTIMATING A RISK OF RELAPSE OF ADDICTIVE BEHAVIOR**

VERFAHREN UND VORRICHTUNG ZUR SCHÄTZUNG EINES RISIKOS DES RÜCKFALLS EINES SUCHTVERHALTENS

PROCÉDÉ ET DISPOSITIF D'ESTIMATION D'UN RISQUE DE RECHUTE D'UN COMPORTEMENT ADDICTIF

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **04.12.2017 SE 1751492**

(43) Date of publication of application:
**14.10.2020 Bulletin 2020/42**

(73) Proprietor: **Kontigo Care AB
753 20 Uppsala (SE)**

(72) Inventors:
• **HÄMÄLÄINEN, Markku
753 30 Uppsala (SE)**

• **ZETTERSTRÖM, Andreas
815 69 Månkarbo (SE)**
• **ANDERSSON, Karl
755 78 Vänge (SE)**

(74) Representative: **AWA Sweden AB
Box 5117
200 71 Malmö (SE)**

(56) References cited:
**EP-A1- 3 091 489        WO-A1-2016/178617
WO-A2-2014/028888     US-A1- 2009 292 180
US-A1- 2013 166 319     US-A1- 2015 339 456
US-A1- 2016 196 389     US-A1- 2017 181 696**

## Description

### Field of invention

The present invention generally relates to addictive disorders and more specifically to methods and devices for estimating a risk of relapse of addictive behavior related to exposure to alcohol.

### Background of the invention

Addictive disorders are common in modern society. Some individuals are addicted to alcohol or other drugs (cocaine, heroin, methamphetamine to mention a few non-limiting examples). Some other individuals are addicted to food consumption and still others are addicted to gaming of various kinds. Smoking is also an addictive behavior that sometimes is seen as a disorder.

Addictive disorders pose a serious threat to modern society. Addiction can take many forms, and one common form of addiction is the intentional use of chemical compounds such as alcohol that intoxicate the user, which causes high costs for health care. Another possible addiction is food related, where a user is either eating excessive resulting in obesity or starving resulting in anorexia. Yet another possible addiction is related to computer gaming, where a user is spending an unhealthy amount of time on computer based games with the potential result of ruining private economy. There are treatment programs for these and other addictive disorders.

One approach to estimate risk for relapse of alcohol dependence has been described in the patent application UA79063 "Method for integrated diagnostics of possibilities of alcohol dependence relapses". The document discloses a method for integrated diagnostics of possibilities of relapse which contains determination of specific and symptomatic syndromologic signs of alcohol dependence. In addition, test monitoring of general psychological quality of life and diagnosis of latent tremor by a laser scanning method is performed. Output of measurements are combined into a score which is indicative of risk for relapse.

Gaming and gambling disorders have relapse patterns that are difficult to manage. One exemplary disclosure on this subject is the report "Retrospective and Prospective Reports of Precipitants to Relapse in Pathological Gambling." by Hodgins, David C. and el-Guebaly, Nady as published in Journal of Consulting and Clinical Psychology, Vol 72(1), Feb 2004, 72-80. http://dx.doi.org/10.1037/0022-006X.72.1.72. This document discloses that before a gaming disorder relapse, participants in the clinical study reported a wide range of moods and emotions with no dominant pattern. Furthermore, men and women also attributed relapses to different causes. The suggestion is that there is no quick fix or single treatment model for gambling relapse. Interestingly, reports of positive moods were as common as negative moods among the participants. This finding stands, according to the authors, in contrast to previous findings that negative moods are the most frequent predictor across a range of addictive behaviors. Another contradictory finding was reported: The reasons for quitting gambling are similar to the reasons for relapse to some degree. All in all, this disclosure describes that the understanding of gaming disorder is still limited, which leads to the conclusion that treatment programs and relapse monitoring have great room for improvement.

In the disclosure "Predictive Modeling of Addiction Lapses in a Mobile Health Application" by Chih and co-authors (as published in J Subst Abuse Treat. 2014 January; 46(1): 29-35.

doi:10.1016/j.jsat.2013.08.004.), a questionnaire-based system is used to predict and reduce relapses of addictive behavior. This is a weekly check-up system suitable for the monitoring of recovery from addiction to alcohol.

WO2016178617 discloses a method and a device for estimating a risk of relapse of addictive behavior. The disclosed method is based on repeated registering of an individual's exposure to an addictive stimulus, and/or answers to a questionnaire, where a risk of relapse is estimated based on a combination of recently received results from these measurements and/or answers, and historic results.

### Related documents

In the published US patent application US 2017/0181696 A1, a method and system for monitoring a user's intoxication are disclosed. A temporal profile is generated as a tool for monitor his/her intoxication.

### Summary of the invention

It is an object of the present disclosure to provide methods and devices for estimating a risk of relapse of addictive behavior. This and other objects are met by embodiments of the invention disclosed in the present application, which is based on the repeated registering and analysis of exposure to stimuli which results in a capability to provide a more complete view of an individual's exposure to stimuli when estimating the risk for relapse, for example during or after treatment of addictive behavior related to said stimuli.

Accordingly, based on the discoveries of the present disclosure, one aspect of the present disclosure provides a method for estimating a risk of relapse of addictive behavior of an individual, the addictive behavior being related to exposure to alcohol, the method comprising the steps of providing a predetermined schedule for the individual to conduct exposure tests, where the exposure test comprises performing a determination of the individual's exposure to alcohol, and the predetermined schedule comprises multiple points in time with time-slots within which said exposure tests should be conducted, requesting the individual to conduct exposure tests according to the predetermined schedule , collecting, with a measurement device, data from the conducted exposure tests, where the collected data comprises measurements of the individual's exposure to alcohol and time-points when the exposure tests were conducted, transmitting the collected data from the measurement device to a central server unit; storing the collected data in the central server unit, evaluating a behavioral pattern related to said individual's compliance to said scheduled exposure tests, based on a determination if said exposure tests are systematically shifted within acceptable time-slots so as to widen the unmonitored time for consuming alcohol in a physically undetectable manner, wherein the behavioral pattern is evaluated based on a Maximum Time Between Exposure Tests, MTBET, where MTBET is the maximum time of all "time between exposure tests" that have been collected during a specific period of time, where "time between exposure tests" is the time between two adjacent exposure tests that both are conducted according to the predetermined schedule, estimating a risk of relapse based on the evaluated behavioral pattern and the measurements of the individual's exposure to alcohol, wherein an elevated MTBET is interpreted as elevated risk for relapse of addictive behavior, and if the estimated risk is greater than a predefined level, transmitting a message to a third party.

Another aspect of the present disclosure provides a server unit configured to estimate a risk of relapse of addictive behavior of an individual, the addictive behavior being related to exposure to alcohol, wherein the server unit is configured to provide a predetermined schedule for the individual to conduct exposure tests, the exposure test comprising performing a measurement of said individual's exposure to alcohol, and the predetermined schedule comprising multiple points in time with time-slots within which the exposure tests should be conducted, request the individual to conduct exposure tests according to the predetermined schedule, receive, from a measurement device, data collected from the exposure tests, the collected data comprising determination of the individual's exposure to alcohol and time-points when the exposure tests were conducted, store the received data, evaluate a behavioral pattern related to said individual's compliance to said scheduled exposure tests, based on a determination if said exposure tests are systematically shifted within acceptable time-slots so as to widen the unmonitored time for consuming alcohol in a physically undetectable manner, wherein the behavioral pattern is evaluated based on a Maximum Time Between Exposure Tests, MTBET, where MTBET is the maximum time of all "time between exposure tests" that have been collected during a specific period of time, where "time between exposure tests" is the time between two adjacent exposure tests that both are conducted according to the predetermined schedule, estimate a risk of relapse based on the evaluated behavioral pattern and said measurements of said individual's exposure to alcohol, wherein an elevated MTBET is interpreted as elevated risk for relapse of addictive behavior, and if the estimated risk is greater than a predefined level, transmit a message to a third party.

Yet another aspect of the present disclosure provides a system configured to estimate a risk of relapse of addictive behavior of an individual, the addictive behavior being related to exposure to an addictive stimulus, wherein the system comprises a server unit as disclosed herein, and a measurement device configured to enable estimation of a risk of relapse of addictive behavior of an individual, the addictive behavior being related to exposure to an addictive stimulus, wherein the measurement device is configured to, at multiple points in time according to a predetermined schedule, perform a measurement of the individual's exposure to the stimulus, and transmit data from the stimulus exposure measurement to a central server unit to enable estimation of a risk of relapse.

## Brief description of the drawings

The embodiments, together with further objects and advantages thereof, may best be understood by making reference to the following description taken together with the accompanying drawings, in which:

Figure 1a is a schematic flow diagram illustrating an example of a method for estimating a risk of relapse of addictive behavior according to an embodiment.

Figure 1b is a schematic flow diagram illustrating optional additional steps of the method of Figure 1a according to an embodiment.

Figure 2 is a schematic diagram illustrating an example of a system configured to estimate a risk of relapse of addictive behavior according to an embodiment.

Figure 3 shows the correlation between average MTBET and PEth according to an example.

Figure 4 shows the highest measured breath alcohol and MTBET per day during one year for an individual according to an example.

Figure 5 shows the highest measured breath alcohol and MTBET per day during about 110 days for an individual according to an example.

Figure 6 shows a decision-tree illustrating the added value of MTBET.

Figure 7 shows a comparison of only AMI and a combination of AMI and MTBET information for an individual during about 110 days according to an example.

Figure 8 shows the distribution of MTBT data collected from patients in clinical trials, where the patients were following predetermined test schedules, according to an example.

## Detailed description of the invention

For the purpose of this application and for clarity, the following definitions are made:
The term "addiction" refers to a state of an individual characterized by compulsive engagement in rewarding stimuli (i.e. stimuli that the brain interprets as intrinsically positive or as something to be approached), despite adverse consequences.

The term "addictive behavior" refers to a behavior which is both rewarding and reinforcing. It may involve any activity, substance, object, or behavior that becomes the major focus of an individual's life and which results in a physical, mental, and/or social withdrawal from normal day to day obligations.

The term "stimulus" refers to an activity, or substance that an individual is addicted to, and that may cause addictive behavior. One non-limiting example of a stimulus is gambling. Another non-limiting stimulus is food and eating. Yet another non-limiting example of a stimulus is an intoxicating chemical compound.

The term "intoxicating chemical compound" refers to a single compound or a combination of multiple compounds capable of intoxicating an individual to a level where the general status of said individual is affected. One non-limiting example of an intoxicating chemical compound is ethanol, more commonly known as alcohol, which is readily available to individuals in wine, beer, spirits and other beverages. Ethanol is intoxicating individuals to a level where many countries have a limit for the allowed amount of ethanol in the blood to drive a car legally. Other intoxicating chemical compounds include, but are not limited to: cannabinoids as for example available in cannabis, caffeine, MDMA (3,4-methylenedioxy-methamphetamine), cocaine, amphetamine, methamphetamine, psilocybin (for example found in "magic mushrooms"), LSD, opiates and opioids, tranquilizers like barbiturates, benzodiazepines and the similar, ketamine, amyl nitrite, mephedrone, mescaline, DMT for example as primary ingredient in ayahuasca, cathine and cathinone (khat), methylphenidate, fentanyl, GHB, ecstasy, narcolepsy medications, sleeping pills, anxiolytics, sedatives, cough suppressants, benzydamine, ephedrine, pseudoephedrine, dimethyltryptamine (DMT), 5-MeO-DMT, theobromine, kavalactones, myristicin, atropine, scopolamine, mitragynine, mitraphylline, 7-hydroxymitragynine, raubasine, valerian, lysergic acid amide (LSA, ergine), ibogaine, arecoline, rauwolscine, yohimbine, corynantheidine, psilocybin, psilocin, bufotenin, ibotenic acid, muscimol, antihistamines including but not limited to diphenhydramine, chlorpheniramine, orphenadrine and hydroxyzine, scopolamine, paracetamol (para-acetylaminophenol), non-steroidal anti-inflammatory drugs (NSAIDs) such as salicylates, hydrocodone, codeine, oxycodone, hydromorphone, carisoprodol, chloral hydrate, diethyl ether, ethchlorvynol, gabapentin, gamma-butyrolactone (GBL, a prodrug to GHB), gamma-hydroxybutyrate (GHB), glutethimide, ketamine, meprobamate, methaqualone, phenibut, pregabalin, propofol, nepetalactone, dimenhydrinate, hyoscyamine, dextromethorphan, dextromethorphan, chlorpheniramine, methoxetamine, phencyclidine, and nitrous oxide, to mention a few examples.

The term "stimuli exposure" refers to an individual becoming exposed to a particular stimulus, either through own will or unintentionally. For example, in cases where the stimulus is an activity such as gambling or eating, "stimuli exposure" refers to engaging in such an activity. As another example, in cases where the stimulus is an intoxicating chemical compound, "stimuli exposure" refers to ingesting, injecting, inhaling, or in any other way introducing the intoxicating chemical into the body of the individual.

The term "stimuli exposure history" refers to the status of an individual in terms of repeatedly measured exposures of one or more stimulus to said individual. Stimuli exposure history may for example contain frequency of exposure, intensity of exposure and duration of exposure. Exposure history may optionally be divided into different timeframes. Some non-limiting examples of timeframes may be: Current stimuli exposure which for example may relate to the timeframe of 1-3 days, short term stimuli exposure history which for example may relate to the timeframe of 1-30 days, medium term stimuli exposure history which for example may relate to the timeframe of 10-100 days, and long term stimuli exposure history which for example may relate to the timeframe of 1-6 months to 1-10 years or even longer.

The term "intoxication history" refers to the status of an individual in terms of repeatedly measured content of one or more intoxicating chemical compounds in said individual. Intoxication history may for example contain frequency of intoxication, intensity of intoxication and duration of intoxication. Intoxication history may optionally be divided into different timeframes. Some non-limiting examples of timeframes may be: Current intoxication which for example may relate to the timeframe of 1-3 days, short term intoxication history which for example may relate to the timeframe of 1-30 days, medium term intoxication history which for example may relate to the timeframe of 10-100 days, and long term intoxication history which for example may relate to the timeframe of 1-6 months to 1-10 years or even longer.

The term "clean" refers to the status of an individual as either being completely unaffected by intoxicating chemical compounds or other addictive stimuli, or being exposed to a certain stimulus to a level which is below a predetermined threshold.

The term "central server" refers to a computer which is available through one or more communication protocols such as the internet.

The term "actuator" refers to actuators suitable for providing feedback to the individual. Examples of such actuators include, but are not limited to, mechanical actuators (e.g. similar to the vibration function of a smartphone), optical actuators (e.g. flashing lamp), audio actuators (e.g. playing a sound or a prerecorded voice message), electrical actuators (e.g. transmitting a small but notable current into the individual through electrodes being in contact with the individual), magnetic actuators, and electrical field generating actuators. Other types of actuators include, but are not limited to, showing a picture or a video on a digital screen attached to the device and releasing a chemical agent with a particular smell.

The term "exposure test" refers to an attempt to determine if an individual has been exposed to any addictive stimuli. One non-limiting example of an exposure test is to measure the level of e.g. alcohol in the breath of an individual. Another non-limiting example of an exposure test is to present a questionnaire to an individual and request answers to questions.

The term "schedule" refers to a series of time-slots in time when an individual is supposed to conduct an exposure test.

The term "time between exposure tests" refers to the time between two adjacent exposure tests that both are conducted within their respective specified time-slots.

The term "maximum time between exposure tests", abbreviated MTBET, refers to the maximum time of all "time between exposure tests" that have been collected during a defined period of time, such as a full day (24 hours) or a full week (7 days).

A "behavioral pattern" refers to an action made by an individual in a repeated manner to satisfy a need, both in cases where the individual intentionally acts and when the action is intentional. One non-limiting example of a behavioral pattern (intentional or unintentional) is to light a cigarette e.g. when exiting a building and going outdoors. Still another non-limiting example of a behavioral pattern (intentional or unintentional) is to repeatedly visit a defined locale (such as a bar, a silent park, a physical exercise facility, or anything else) so as to satisfy a need (such as social interaction or quiet loneliness to mention two very different examples). Still another non-limiting example of a behavioral pattern is to intentionally delay or ignore a morning test of breath alcohol, so as to avoid detection of having consumed alcohol in the evening. Still one non-limiting example of a behavioral pattern is that a substance use disorder is replaced by another use dependence (e.g. gambling, food, sex etc.).

The present invention provides a method to aid in determining if an individual is at risk for a relapse of an addictive behavior. In other words, the invention comprises a method for improved identification of relapse, for example during and after treatment for addictive disorders. The present invention also provides a device suitable for use in the method. The basic principle of the invention is to provide an individual with a predetermined schedule with a series of time-slots within which exposure tests should be conducted, to record the individual's exposure to addictive stimuli in these time-slots, to determine behavioral patterns related to compliance to the scheduled exposure tests from a time-series of conducted exposure tests, and to determine, partly based on the recorded stimuli exposure, and partly based on behavioral patterns, if an individual is at risk for a near-future relapse of addictive behavior. Upon detection of increased risk for relapse, health care providers or other third parties can be alerted so as to support the individual being treated in stopping the relapse.

The individual does typically have an acceptable time-span to conduct an exposure test according to the predetermined schedule, and can intentionally or subconsciously choose to conduct exposure tests systematically shifted within acceptable time-slots. Surprisingly, systematic trends in how the actual time-point for conducting a requested exposure test, even within acceptable time-span, have been discovered to correlate well with elevated risk for relapse. Hence, such behavioral patterns can support physical measurements and/or questionnaire answers in calculating an estimate for risk for relapse. US20160196389 A1 discloses a method for providing patient indications to an entity in the healthcare field. In this method, a behavioral dataset is generated based on a log of the use of a communication application executing on a mobile device, and this behavioral dataset is used for determining if a patient has a particular indication. The method is used to passively monitor the patient interacting with the mobile device in order to enable detection of deviations from patterns in behavior, such as mobility behavior or communication behavior, e.g. how and at which frequency the patient communicates with other individuals through phone calls etc.

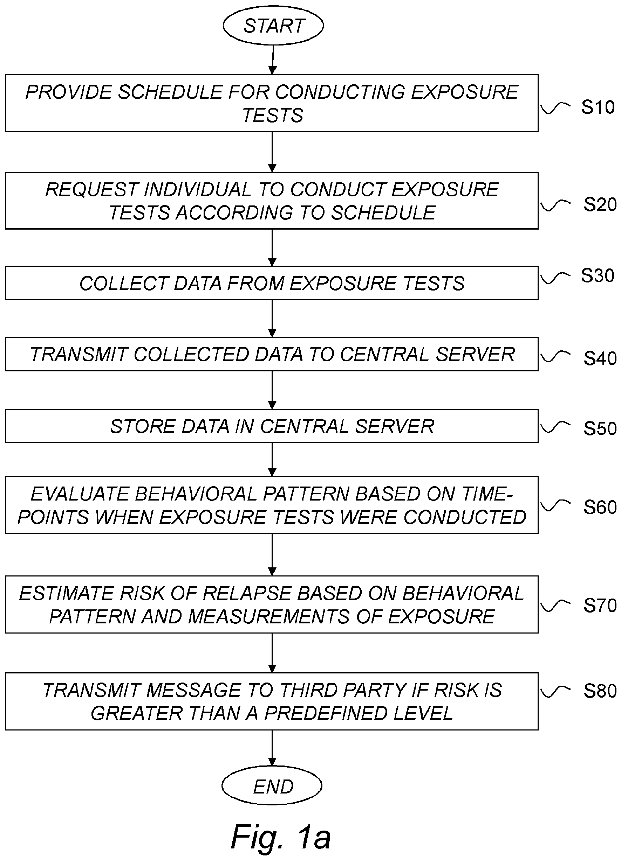

Figure 1a is a schematic flow diagram illustrating an example of a method for estimating a risk of relapse of addictive behavior of an individual according to an embodiment of the present invention, where the addictive behavior is related to exposure to an addictive stimulus. The method comprises:

- providing S10 a predetermined schedule for an individual to conduct exposure tests, where an exposure test comprises performing a measurement of the individual's exposure to the addictive stimulus, and the predetermined schedule comprises multiple points in time with time-slots within which exposure tests should be conducted;
- requesting S20 the individual to conduct exposure tests according to the predetermined schedule;
- collecting S30, with a measurement device, data from conducted exposure tests, where the collected data comprises measurements of the individual's exposure to the addictive stimulus and time-points when the exposure tests were conducted;
- transmitting S40 the collected data from the measurement device to a central server unit;
- storing S50 the collected data in the central server unit;
- evaluating S60 a behavioral pattern of how the exposure tests were conducted, based on at least the time-points when the exposure tests were conducted;
- estimating S70 a risk of relapse based on the evaluated behavioral pattern and the measurements of the individual's exposure to the addictive stimulus; and
- if the estimated risk is greater than a predefined level, transmitting S80 a message to a third party.

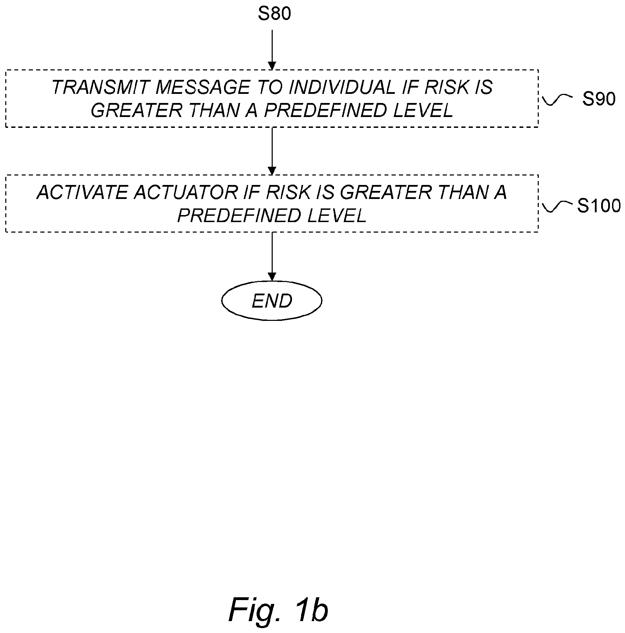

Figure 1b is a schematic flow diagram illustrating optional additional steps of the method of Figure 1a. In this embodiment, the method further comprises a step S90 of transmitting a message to the individual, and/or a step S100 of activating an actuator in the measurement device, if the estimated risk is greater than the predefined level.

The behavioral pattern is related to the individual's compliance to the scheduled exposure tests, i.e. whether the individual conducted the exposure tests according to the predetermined schedule. The behavioral pattern is related to conditions of exposure tests that are conducted <u>within</u> acceptable time-spans/slots as defined in the predetermined schedule.

In an embodiment the behavioral pattern is evaluated based on time between conducted exposure tests, and/or location where exposure tests have been conducted, and/or time to complete an exposure test.

The behavioral pattern is evaluated based on a Maximum Time Between Exposure Tests, MTBET, where MTBET is the maximum time of all "time between exposure tests" that have been collected during a specific period of time, where "time between exposure tests" is the time between two adjacent exposure tests that both are conducted according to the predetermined schedule. In a particular embodiment, an exposure test also comprises answering a questionnaire provided in the measurement device, and the collected data from the conducted exposure tests also comprises the individual's questionnaire answers. In this embodiment the risk of relapse is estimated also based on the questionnaire answers.

In some embodiments the estimated risk for relapse can be used also to determine a new point in time for requesting the individual to conduct an exposure test, in addition to the already scheduled exposure tests. For example, if the patient skips one of the scheduled tests a new test can be triggered.

The method may also be understood as comprising:

- Supplying an individual with a measurement device capable of:

    ○ Measuring the exposure to stimuli
    ○ Optionally, presenting questions to a user and collecting answers
    ○ Communicating with a central server unit.

- Said central server unit requesting said individual to use the device at multiple points in time according to a predetermined schedule and storing the results in the central server unit.
- Using a time-series of conducted exposure tests to evaluate behavioral patterns.
- Upon the central server unit receiving a result, estimating the risk for relapse of addictive behavior and making the estimate available to a defined third party such as a health care provider or a family member.

In some embodiments a request for questionnaire answers is made at the same point in time as a request for a measurement, but it is equally possible to request only a measurement at a certain point in time, or to request only questionnaire answers at a certain point in time. Thus, a request for a measurement and a request for questionnaire answers can be made simultaneously or at different points in time, and with different time intervals or with the same time intervals, if desired.

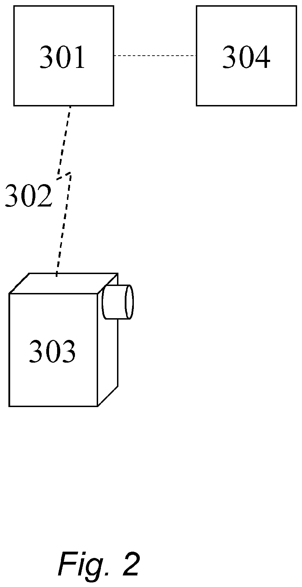

Figure 2 is a schematic diagram illustrating an example of a system configured to estimate a risk of relapse of addictive behavior according to an embodiment. The system comprises a server unit 301 and a measurement device 303, communicating through a wireless connection 302. The central server unit 301 requests an individual to conduct exposure tests according to a predetermined schedule, using the measurement device 303. The measurement device 303 then reports back the result of the exposure tests to the central server unit 301 using the wireless connection 302, either the measured values or that the test was not conducted as requested. Upon the central server unit 301 receiving a result, an estimate of the risk for relapse of addictive behavior is calculated. If the risk for relapse exceeds a predetermined threshold, the central server unit attempts to notify a third party recipient 304, and possibly also the individual (either using an encouraging message or a disturbing action, both for the purpose to disrupt the trend and stop the anticipated relapse). In the latter case the device 303 itself is preferably used. If the risk for relapse is below the predetermined threshold, no action is taken. The estimated risk for relapse can further be used to determine a new point in time when a measurement should be requested, in addition to the already scheduled tests.

The measurement device 303 shown in figure 2 is configured to enable estimation of a risk of relapse of addictive behavior of an individual, where the addictive behavior is related to exposure to an addictive stimulus. In an embodiment, the measurement device 303 is configured to do the following at multiple points in time, according to the predetermined schedule:

- perform a measurement of the individual's exposure to the stimulus, and optionally provide a questionnaire to the individual and collect the individual's questionnaire answers, and
- transmit data from the stimulus exposure measurement and optionally the questionnaire answers to a central server unit 301 to enable estimation of a risk of relapse.

Thus, the measurement device is capable of measuring the exposure to stimuli, possibly presenting questions to a user and collecting answers, and of communicating with a central server. The measurement device may be one physical unit or may comprise several physical units. One non-limiting example of a measurement device is a two unit device comprising a breathalyzer capable of monitoring alcohol content in an individual's breath and one smart phone capable of presenting questions and communicating with a central server. The two units in this case can be connected using a cable or a short-range communication protocol such as Bluetooth, to mention two non-limiting examples. A non-limiting example of a measurement device comprising one physical unit is a balance capable of measuring the weight of an individual, where the balance is connected to a small computer panel which is capable of presenting questions and collecting answers, and which is capable of communicating with a central server. Still another non-limiting example is a smartphone which is monitoring the user's engagement in on-line gambling. The smartphone is further capable of presenting questions and collecting answers, and which is capable of communicating with a central server. Yet another non-limiting example is the use of wearable sensors (i.e. sensors that are essentially attached to the body of a human, such as a bracelet or sensors embedded into clothes) suitable for detecting a body state which in turn can be linked to stimuli exposure. As an example, one possibility could be to register degree of motion and pulse, where low degree of motion combined with high pulse could e.g. be related to computer gaming but a high degree of motion combined with high pulse excludes computer gaming as a cause for the detected body state. Furthermore, a device may include actuators suitable for providing feedback to the individual. Examples of such actuators include, but are not limited to, mechanical actuators (e.g. similar to the vibration function of a smartphone), optical actuators (e.g. flashing lamp), audio actuators (e.g. playing a sound or a prerecorded voice message), electrical actuators (e.g. transmitting a small but notable current into the individual through electrodes being in contact with the individual), magnetic actuators, and electrical field generating actuators. Other types of actuators include, but are not limited to, showing a picture or a video on a digital screen attached to the device and releasing a chemical agent with a particular smell.

When activating an actuator, it is possible to design the actuator so that it is acting on or near known trigger points on the body. The so-called NADA acupressure points have been used in management of addictive disorders, as discussed for the case of smoking in the report "Effect of self-administered auricular acupressure on smoking cessation--a pilot study" by Leung and co-authors as published in BMC Complement Altern Med. 2012 Feb 28;12:11. doi: 10.1186/1472-6882-12-11. This means, for example, that one or more actuators providing physical pressure on known trigger points can deliver acupressure treatment at points in time where the risk for relapse is elevated. Another non-limiting alternative is to stimulate known trigger points used in acupressure or acupuncture with a small electrical current, so as to deliver treatment at points in time where the risk for relapse is elevated.

The server unit 301 shown in figure 2 is configured to estimate a risk of relapse of addictive behavior of an individual, where the addictive behavior is related to exposure to an addictive stimulus. The server unit 301 is configured to do the following:

- provide a predetermined schedule for an individual to conduct exposure tests, where an exposure test comprises performing a measurement of the individual's exposure to the addictive stimulus, and the predetermined schedule comprises multiple points in time with time-slots within which exposure tests should be conducted;

- request the individual to conduct exposure tests according to the predetermined schedule;
- receive, from a measurement device 303, data collected from the exposure tests, where the collected data comprises measurements of the individual's exposure to the addictive stimulus and time-points when the exposure tests were conducted;
- store the received data;
- evaluate a behavioral pattern of how the exposure tests were conducted, based on at least the time-points when the exposure tests were conducted;
- estimate a risk of relapse based on the evaluated behavioral pattern and the measurements of the individual's exposure to the addictive stimulus; and
- if the estimated risk is greater than a predefined level, transmit a message to a third party 304.

In an embodiment the behavioral pattern is related to the individual's compliance to the scheduled exposure tests, i.e. whether the individual conducted the exposure tests according to the predetermined schedule. In a particular embodiment the behavioral pattern is related to conditions of exposure tests that are conducted within acceptable time-spans/slots as defined in the predetermined schedule.

In a particular embodiment, the collected data from the conducted exposure tests also comprises the individual's answers to questionnaires. In this embodiment the risk of relapse is estimated also based on the questionnaire answers.

In some embodiments the server 301 is further configured to determine a new point in time for requesting the individual to conduct an exposure test, in addition to the already scheduled exposure tests, based on the estimated risk.

One important feature of the invention is that the central server unit is requesting the individual to make exposure tests mainly according to a predetermined schedule. This makes it difficult for the individual to hide intentional exposure to stimuli, because the time-points for exposure tests are out of the individual's control. The request as such can, for example, be communicated through the measurement device as a sound or a lamp being switch on. Another non-limiting alternative is to make the central server unit send a short text message (SMS) to the individual's cell phone. Yet another non-limiting alternative is to make the central server unit call the individual and play a prerecorded phrase which tells the individual that a measurement should be conducted. Still another non-limiting alternative is to make the central server unit send an e-mail to the individual.

The central server attempts to determine the likelihood that the individual is at risk for relapse each time new data is submitted to the central server. The server is then determining if the individual is at elevated risk and, optionally, a suitable time point for the next exposure test request to the individual. If the central server determines that the individual is, with a high likelihood, at risk for relapse, for example if the risk is above a predefined threshold, a message can be sent to a third party, such as a family member, a health care provider, employer, law enforcement or any other party. This message can be transmitted using the internet or a GSM cell phone network or any other similar network. Additionally, the central server may, if the risk for relapse is exceeding a predetermined level, instruct the device to provide feedback to the individual, so as to attempt to disturb the pattern indicative of relapse and thereby possibly prevent relapse from advancing or even occurring. As an example, the provided feedback may be transmitted as playing a prerecorded message, or sending a short text message (SMS) to the individual's cell phone, with an encouraging content. As another example, the feedback may be of annoying character, such as vibrating the device or playing an annoying alarm.

The measurement device should preferably contain some kind of feature that identifies the individual who is actually using the device. If such a feature is not available, it would be possible for an individual to ask a clean friend to operate the measurement device at times when a measurement is requested. One possible method for identifying the individual who is currently using the measurement device is to let the device take a photo of the operator during the measurement and transfer the photo to the central server together with the obtained result. In case of suspicions regarding an individual attempting to falsify measurements, such photos could be reviewed. Other possible methods for identifying the individual who is operating the measurement device includes, but is not limited to, conducting a retinal or iris scan during measurement, analyzing a voiceprint of the individual or reading a fingerprint during measurement. As an example, a fingerprint reader or other suitable device for identifying the individual could be attached to the measurement device.

The central server unit is typically a computer which comprises a program capable of regularly, or with irregular time intervals, requesting an individual to conduct exposure tests, storing measurement results, and estimating if present data is indicative of the individual being clean. The purpose of repeatedly requesting the individual to make an exposure test is to collect a time-line of stimuli exposure data, such as intoxication data. Such a stimuli exposure history can reveal onset patterns, frequency, duration and intensity of stimuli exposure. Such information is valuable when assessing the likelihood that an individual is clean. For example, if stimuli exposure history indicates that an individual is highly exposed to stimuli approximately once per month with onset in the evening, but the duration is only one day, it may be sufficiently accurate to rely on an evening measurement previous day until the afternoon present day. As another example, if a different individual has a stimuli exposure history which indicates frequent stimuli exposures or intoxications at mild intensity during 3-5 days, a new measurement may be requested multiple times per day.

A key feature of the present invention is the ability to estimate the likelihood of an individual being clean based on historic data. In addition to actual measurements of stimulus (for example alcohol in an individual's breath), answers to questionnaires, and missed tests, the behavioral pattern in how scheduled tests are actually performed correlate to relapse risk. The behavioral pattern may be related e.g. to compliance to the scheduled exposure tests, i.e. that the individual actually performs the tests as scheduled, but also to the manner in which the tests are performed, even when within requested scheduling. As a non-limiting example, an individual with an alcohol use disorder who at a certain point in time becomes at elevated risk for relapse may from that time and onwards start to shift the late evening exposure test as early as acceptable, and may also conduct the morning exposure test as late as possible, so as to widen the unmonitored time and allow for consuming alcohol in a physically undetectable manner. By monitoring behavioral patterns, such attempts become visible and useful indicators in the evaluation of risk for relapse. An individual who at a certain point in time becomes at elevated risk for relapse may furthermore intentionally attempt to adjust the scheduling so that longer periods of time are unmonitored: for example by claiming morning stress and requesting the schedule to move the morning exposure test forward in time a seemingly short time, such as for example 0.25, 0.5, 0.75, 1, 1.5, 2, 2.5, 3, 3.5 or 4 hours or anything similar.

As a non-limiting example, one simple yet powerful behavioral indicator is "time between exposure tests", which is the amount of time that has passed since the most recent accepted exposure test. A day can be represented by the "maximum time between exposure tests" (MTBET), which is the maximum time between exposure tests of all available time between exposure tests in a given day. MTBET has been shown to correlate well to risk for relapse. Another non-limiting example is change in the average total time required to complete an exposure test.

Analysis of behavioral patterns may provide indications to whether an individual is trying to tamper with the measurements/tests. Such attempts may be discovered by evaluating quality parameters of the measurements. A non-limiting example of such a quality parameter is logging motion patterns of the device during measurement/test and indicating when the motion pattern during a particular measurement/test is deviating significantly from the most common motion patterns. Exposure to an addictive stimulus may also be detected by other means than the actual measurements, e.g. by analyzing how the individual handles the measurement device, for example via a gyro sensor in the device.

It is possible and desirable to use many different underlying methods for determining if the stimuli exposure history, e.g. intoxication history, and exposure test behavioral patterns are indicative of an individual being clean. One non-limiting method is to make a weighted average of the measurement results related to stimuli exposure the recent year, where newer measurements are given higher weight than older. Should an individual be confirmed exposed to stimuli at one point in time, this exposure event will have high impact on the cleanness estimation shortly after, but will gradually reduce in impact as time passes. Another possible method is to extract a short term stimuli exposure history which relates e.g. to the previous month and a long term stimuli exposure history that relates e.g. to approximately the recent year or years, and evaluate if both the short term and the long term history are indicative of cleanness. In the short term assessment, the onset of a relapse can be captured, while as in the long term assessment, the change of the stimuli exposure habit of the individual can be monitored. This makes it possible to determine if a present stimuli exposure event is likely an accident (if the long term history suggests that the individual is staying clean most of the time) or if it likely is part of a pattern suggesting a larger relapse. Such a procedure can be discussed in more detail in the non-limiting context of use of intoxicating chemicals such as alcohol. One possible non-limiting example of a method for combining short term intoxication history with medium term or long term intoxication history for the purpose of determining the cleanness of an individual is the following. First, a short term intoxication (STI) value is calculated. This STI value can be constructed in many different ways, but one possible definition is the following:

STI = ((mild intoxication days the recent 30 days) + 3*(severe intoxication days the recent 30 days) + (average MTBET the recent 30 days)) / 30

The expression "mild intoxication day" could e.g. refer to a day when a measured value of an intoxicating chemical compound above but near the value for confirming intoxication is registered. "Severe intoxication day" could e.g. refer to a day when a measured value of an intoxicating chemical compound is much higher than the value for confirming intoxication is registered. For example, for alcohol, the limit for confirming intoxication could e.g. be "detection limit of the instrument" or it could be "0.2 ‰ Blood alcohol content" and the limit for considering intoxication to be severe could e.g. be "0.5 ‰ Blood alcohol content".

Second, a medium term intoxication (MTI) value can be calculated in a similar manner. One possible definition is the following:

$$MTI = (STI[30]*3 + STI[60]*2 + STI[90]*1) / 6$$

Wherein STI[x] represents the historic STI value at x days before the day of calculating the MTI value, so that STI[1] represents the STI value of yesterday, and STI[2] represents the STI value of the day before yesterday. In plain words, MTI could e.g. be defined as the weighted sum of three historic STI values (in this example one month ago, two months ago and three months ago) where the most recent historic STI value is given higher weight and the oldest STI value is given lowest weight. A long term intoxication (LTI) value can be calculated in still a similar manner, but covering a time period greater than the MTI value (> 3 months in this example). Similar short-term, medium term and long term indicators can be designed also for other stimuli than intoxicating compounds.

Another key feature of the present invention is the ability to investigate the user mood, stamina, and the like through the use of questionnaires. As an optional feature, since the questionnaire is preferably managed through a computer, it is not only the answers to the asked questions (Questionnaire Answers, QA) that may be important, but also how the answers were delivered (Questionnaire Motorics, QM). For example, in a questionnaire that is related to mood, the time spent to answer questions is likely longer at depressed mood than in good mood. Hence, the answers provided to the mood-related questions can be cross checked to the time spent on answering the questions so as to gather both conscious and motoric information on the same subject for the purpose of producing a more reliable estimate of the true mood of the individual. In a similar manner, if a questionnaire is related to alcohol consumption, the speed and motoric precision will probably be reduced also at low level intoxication. This means that if a multiple-choice question is presented, and time to answer combined with the distance between where on the screen the individual indicates the answer as compared to the position of the indicators for the multiple choice answers will reveal the speed and motoric precision of the individual. When combining the conscious and motoric information on the same subject for the purpose of producing a more reliable estimate of the true condition of the individual. It is beneficial to gather baseline information about each individual to make possible comparisons of current Questionnaire Answers (QA) and current Questionnaire Motorics (QM) so that deviations from the normal state of an individual can be evaluated.

By combining intoxication measures of different time-frames like e.g. STI, MTI, LTI, QA, QM, MTBET and/or other data a reliable measure of the likelihood of being at risk for relapse can be created. Other data includes, but is not limited to, time of day, day of week, type of day (e.g. holiday, salary day etc.), result of the previous measurement, elapsed time since the previous measurement, number of measurements that have been missed in the recent past, and similar.

In some cases it may, for pedagogic reasons in relation an individual suffering from addictive disorder, be favorable to present an intoxication index as a sobriety index. A sobriety index is always negatively correlated with an intoxication index.

It is also possible to supplement the method with a series of other functions, including but not limited to, monitoring that the measurement device (and hence presumably the individual) has been in close proximity to a predefined geographic position at a predetermined time point (such as meetings with a therapist or meetings with support groups) through use of GPS (global positioning system).

The questionnaire that may for example be embedded in a hand held device, such as a smartphone, and can be configured to at different frequencies (e.g. at least once per week, more preferably once or twice per day, and in extreme cases once per hour during daytime) ask an individual questions that are related to stimuli exposure or that reveal increased risk for the individual exposing himself or herself. Suitable questionnaires may be selected from those available and validated, such as questionnaires to monitor addiction (where AUDIT is one non-limiting example) and psychological status (where MADRS is one non-limiting example). Questionnaires may also cover areas like daily mood and exercise of routines (work, school, therapy), motivation, eating habits, sleeping habits, engagement and self-efficacy, to mention a few non-limiting examples. These data can in the same way as measurements of stimuli exposure be compressed and compiled into short, medium and long term risk factors (SRF, MRF, LRF) useful for estimation of the risk of stimuli exposure, where SRF, MRF and LRF may be used alone or in combination. For example, if the motivation level to be clean is low and the daily mood is poor the risk of self-inflicted stimuli exposure is usually higher and therefore measurements and questionnaires should be performed more frequently.

The actual measurement results related to intoxication can potentially be combined with a range of additional data (some of which are exemplified above, and potentially compressed into variables like SRF, MRF, and LRF), in the process of determining if an individual is likely to be clean or not.

The present invention provides a non-transitory computer readable medium comprising instructions for causing a computer to perform particular steps of the above-described method, such as the estimation of if an individual is likely to be clean which are typically conducted in the central server computer. Furthermore, the measurement device may contain a non-transitory computer readable medium comprising instructions for causing a computer to perform parts of the procedures related to the direct or indirect measurement of stimuli exposure, such as measuring the quantity of a chemical substance, measuring the motoric reaction pattern of an individual, measuring a physical property such as weight of an individual, to mention a few non-limiting examples.

An example of a suitable type of measurement device comprises an apparatus capable of measuring breath alcohol content by analyzing the exhaled air of an individual, where breath alcohol level is a surrogate for blood alcohol level. Such devices are commonly known as breathalyzers. One possibility is use a breathalyzer of type Kontigo tripleA. This device measures the Breath Alcohol (BrAC) Level of an individual using a sampling pump and a fuel cell which converts alcohol content to an electric signal, said signal being dependent on the alcohol concentration. The breath sample is taken when the individual has delivered more than 1.2 liters of air directly from his/her lungs. The electric signal is processed and converted to digital form in a microcontroller and is then compared to calibration values to allow transformation to a corresponding BrAC-level in mg/L. When the BrAC-level has been calculated it can then be transmitted to a connected host smart phone which in turn can deliver the value to a central server unit. Before, during and after the measurement the actual state is visualized to the user with light emitting diode indications in various colors. The Kontigo tripleA device is further equipped with a camera which is depicting the individual during the measurement, so as to provide evidence that it is a particular individual operating the device. This above described example of a measurement device hence comprises two parts: one part that contains a breath analyzer and one other part that is a regular smart phone capable of presenting questions and collecting answers, and communicating with both the breath analyzer and central server units. By dividing the measurement device into two parts where one, the smart phone, is a regular existing product the cost for the measurement device is reduced.

It is important to mention that for a measurement device intended to quantify an intoxicating chemical compound, it is not necessary to measure the exact intoxicating chemical compound. It is in some cases possible to measure a metabolite or a catabolite that is related to the intoxicating compound. The measurement of ketone which is related to alcohol consumption is one such indirect measurement that is capable of determining if an individual has consumed alcohol. Furthermore, measurement devices that conduct the measurement of one or more intoxicating chemical compounds are typically using a non-invasive sample specimen. Examples of non-invasive sample specimens include, but are not limited to, analysis of urine, feces, sweat, saliva, and exhaled air.

The present invention makes it possible to use stimuli exposure history to determine if a health care provider should follow up an individual with known addiction behavior. It is well known that in the care of alcoholics, drug addicts, and individuals with similar diseases or disorders, there is a high frequency of relapse after treatment. As a particular example, in the care of alcoholics it has been reported that even after treatment, the average short-term abstinence rates is only approximately 40% as discussed in the report "Rates and predictors of relapse after natural and treated remission from alcohol use disorders" by RH Moos and BS Moos as published in Addiction. Feb 2006; 101(2): 212-222. This means that the society has a lot to benefit from improved care of alcoholics after the actual treatment has taken place. The present invention can be applied as a monitoring tool where the intoxication history and behavioral pattern is captured and stored, and in cases where an individual is determined to be intoxicated a message can be sent to the health care provider. The present invention is thus capturing relapse of disease in an early stage, which allows health care providers, family members, and other related individuals to become informed so as to attempt to interrupt the relapse. The present invention may, when used as a monitoring tool, be supplemented with a series of other functions, such as support e.g. in the form of encouraging text messages or e-mails, possibly synchronized with times or geographic locations where risk for relapse is high (for example Friday evening in a restaurant area). In practice, the human mind is limited in its ability to combine a large number of input data for concluding if an individual is at elevated risk for a condition. This means that in a system where individuals are continuously monitored, the human mind is practically incapable of capturing small trends in data that may be statistically correlated to risk. Hence, this invention provides a decision-maker (such as a health care professional or a family member, to mention two non-limiting examples) with information that is virtually impossible for the human mind to compile into useful facts or indications and which in particular goes beyond mere aggregation of input data. Accurate and reliable information is essential for the decision-maker to exercise his or her task of making decisions related to the individual: Should for example an individual at elevated risk for relapse of addictive behavior be subjected to immediate care (or prescribed medication, or confined in space, or anything similar), potentially against the individual's will? Only with access to accurate and reliable information, the decision maker can make such a difficult decision with confidence. In other words, mastering the technical task of interrupting a suspected relapse is greatly improved through use of the present invention where reliable information is provided to the decision-maker.

Addictive behavior need not be linked to a chemical compound. An individual may become addicted to e.g. gambling, and with the availability of a multitude of on-line gambling web-sites it may be difficult for an individual to stay clean from gambling. Aspects of pathological gambling is discussed in the report "Retrospective and Prospective Reports of Precipitants to Relapse in Pathological Gambling." by Hodgins, David C. and el-Guebaly, Nady as published in Journal of Consulting and Clinical Psychology, Vol 72(1), Feb 2004, 72-80. http://dx.doi.org/10.1037/0022-006X.72.1.72. Another example is pathological relationships to food, leading to an un-healthy situation (such as anorexia or obesity).

Below are some non-limiting examples of implementations of the invention described.

Example 1

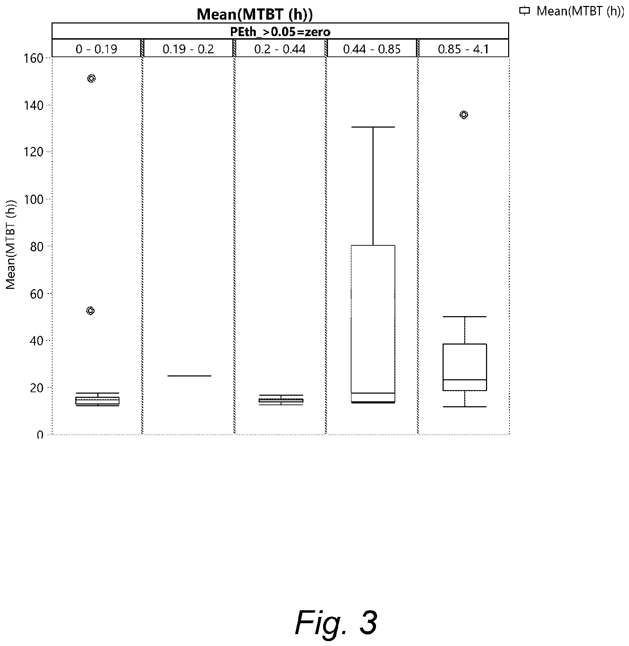

This example shows that it is possible to correlate if an individual with a confirmed elevated value of Phosphatidyl ethanol (PEth) also has an elevated Maximum Time Between Exposure Tests MTBET. It is well known that Phosphatidyl ethanol (PEth) is the golden standard of monitoring medium and long term sobriety of patients with alcohol use disorder, as discussed in "Monitoring of the alcohol biomarkers PEth, CDT and EtG/EtS in an outpatient treatment setting." as published by Helander and co-authors in Alcohol Alcohol. 2012 Sep-Oct;47(5):552-7. doi: 10.1093/alcalc/ags065. PEth is 100% selective and has a half-life of 4-12 days. Figure 3 shows the correlation between weekly average MTBET measured in hours and PEth for 17 patients where in total 50 PEth measurements were conducted. The figure shows the distribution as a box-plot of the mean of MTBET during a week for four levels of actual PEth values. In this example, MTBET was defined in the following manner:

If max TBET < 12 h then MTBET = max TBET during a full calendar day (24h).

If max TBET $\geq$ 12 h then MTBET = max TBET during a full calendar day (24h) + (max TBET - 12)*Penalty.

The Penalty applied for extended MTBET adds weight to MTBET so that each additional hour of "time between tests" beyond 12 hours is stronger, typically twice as strong, in terms of contribution to MTBET.

The correlation between MTBET and PEth is significant and shows that a behavioral change reflected in increased time between tests is related to a verified increase in alcohol use. This means that it is confirmed that MTBET is quality assured as an indicator for alcohol use in alcohol use disorder patients.

Example 2

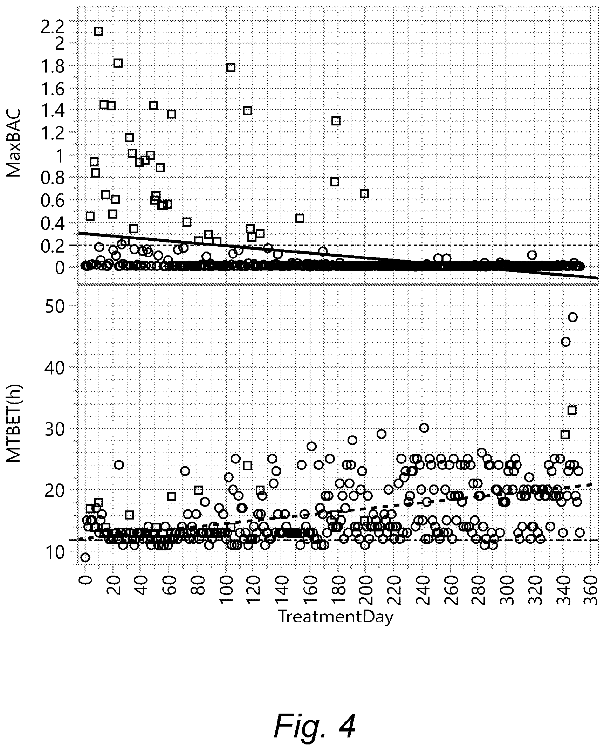

It is a common pattern in addictive disorders that the patient tries to hide a lapse/relapse. One way to hide drinking for a patient with alcohol use disorder is to increase the time between tests - usually the time between the last measurement of the day and the first measurement of the day after. This can be done by performing the last test in the evening as early as possible and the first test in the morning as late as possible - this in compliance with agreed test scheduling but gliding within test windows. Figure 4 shows a patient (A) with the gliding window type of behavioral change where secret drinking was verified with the biomarker PEth. The figure shows the highest measured breath alcohol per day during about one year and the MTBET per day during about one year for patient A. The patient was part of a continuous alcohol use monitoring using a breathalyzer (Kontigo TripleA, Kontigo Care AB, Uppsala Sweden) 2-4 times per day. MTBET was calculated as described in Example 1.

For patient A, the number of tests where alcohol use was verified with breathalyzer (squares, above the dotted threshold of 0.02% blood alcohol level) decrease significantly over time (Fig 4, upper portion). However, this positive change was paralleled by an increased MTBET related to both gliding within windows and omissions of tests leading to a trend with significantly longer MTBET with time (Figure 4, lower portion). Patient A had the highest PEth value in the end of treatment (1 $\mu$mol/L) which verified that MTBET, for this patient, is a predictive tool for identification of behavioral change related to secret drinking. If only results from the Breathalyzer test was used for decision making the caregiver would have inaccurately concluded that the patient has been abstinent for the last ~4 months of the treatment.

Example 3

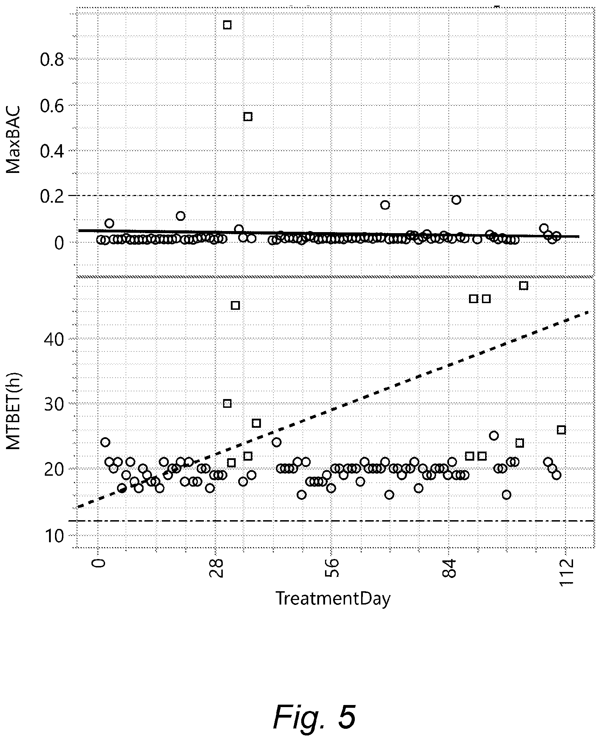

As discussed in Example 2, it is a common pattern in addictive disorders that the patient tries to hide a lapse/relapse. One way to hide drinking for a patient with alcohol use disorder is to increase the time between tests by intentionally selecting a test schedule that allows for infrequent tests with wide acceptable windows. Figure 5 shows a patient (B) with this type of schedule where secret drinking was verified with the biomarker PEth. The figure shows the highest measured breath alcohol per day during about 110 days and the MTBET per day during about 110 days for patient B. The patient was part of a continuous alcohol use monitoring using a breathalyzer (Kontigo TripleA, Kontigo Care AB, Uppsala Sweden) 2-4 times per day. MTBET was calculated as described in Example 1.

For patient B (Fig 5) only 2 days with verified alcohol use (squares) was detected and test compliance was excellent during the first 3 months of the treatment with the exception of one relapse during week 5-6, (diamonds) = all measurements omitted that day. However the patient optimized misuse of the time window of the 2 tests per day schedule having a median MTBET of 20 h. One extra hour in increased MTBET, if used to hide drinking, corresponds to a combustion of 0.01-0.02 % of blood alcohol. The 20 h MTBET was a result of performing the first test before lunch and the second test in early afternoon. A typical MTBET median value is 9-13 hours. The systematically elevated MTBET-value indicated the presence of secret drinking (which was later confirmed) due to gliding within test windows. Test window gliding was presumably conducted to avoid easily detected omission of tests. The schedule used in this case with a large window for each test enabled concealed heavy drinking which was verified by elevated PEth-values (~0.8 $\mu$mol/L).

Example 4

A method which combines results from breathalyzer tests and statistics of omitted tests into an addiction recovery/monitoring index was disclosed in WO2016178617. The addiction recovery/ monitoring index as disclosed in WO2016178617 is denoted AMI (Addiction Monitoring Index) in the following. The AMI is composed of the actual measured blood alcohol values (typically requested three times per day) combined with recent missed measurements. Unwanted results (elevated blood alcohol level or missed measurement) were given additional penalty if occurring evening or morning, and if occurring consecutively. In schematic equation form, AMI was calculated based on "raw Addiction Monitoring Index" (rawAMI) essentially according to

$$rawAMI = 100 - (A) * [BAC] - C*[missedBAC] - D * [consecutive\ penalty]$$

where A, B, C, and D are coefficients or weights, [BAC] is the most recent blood alcohol level measurement (expressed in g/L), [missedBAC] is 1 if the most recent request for measurement of blood alcohol was not conducted within the stipulated time-frame and 0 otherwise, and [consecutive penalty] is 1 if both the most recent blood alcohol measurement and the second most recent blood alcohol measurement both had either elevated blood alcohol level or were not conducted within the stipulated time-frame (and 0 otherwise). The sum of the coefficients (A + B) is approximately 40-60 divided by the blood alcohol legal limit for driving a car. The coefficient C is approximately 40-60, and the coefficient D is approximately 100-C. Addiction Monitoring Index AMI is calculated as the lowest value of rawAMI obtained during a day.

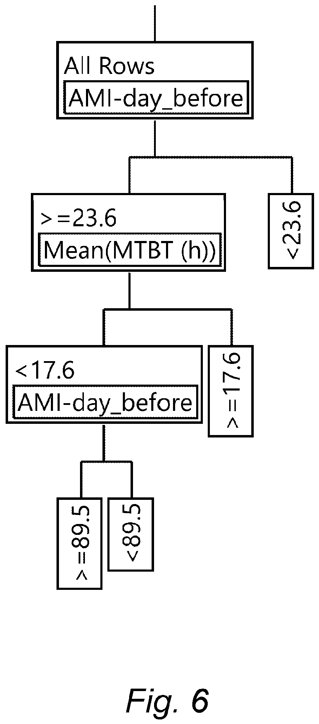

To test if MTBET, as defined in Example 1, added information above what already is included in AMI a decision tree model was built where PEth level was used as a response depicting the state of addiction (Figure 6).

The decision tree started with a split using a low AMI-level of 23 (patients with extreme alcohol consumption giving high PEth and low AMI), followed by a split based on 17.6 h MTBET. This time point around 17 h is interesting because it includes the typical window gliding (morning/evening) and omission of at least an evening or morning test giving in both cases a MTBET values above 17 hours. The AMI did not include window gliding into the model. In the third split data is again split using AMI 89.5 indicating that you need a combination of a MTBET below 17h and an ARI above 90 to have a very low PEth-value and to be considered to be reasonably sober. This simple model explains 52% of the variation in PEth and verifies that MTBET add significant information on the sobriety of the patients.

Example 5

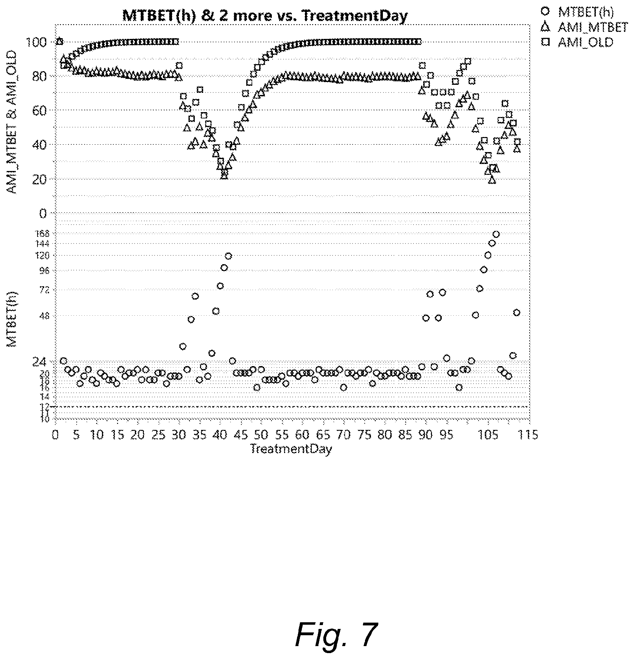

Previously disclosed methods, such as the method disclosed in WO2016178617, for monitoring addiction do not take into account window gliding and had discrete values as a penalty for missed exposure tests. By supplementing such methods, for example the AMI method as discussed in Example 4, with a test compliance component based on MTBET, the [AMI + MTBET] composite value increases the performance and accuracy of addiction monitoring. Figure 7 shows a comparison of the AMI and the [AMI+MTBET] composite value for one individual during about 110 days. Misuse of a test window by extreme gliding, which was previously undetected, will in a composite value result in lower [AMI+MTBET]-values (Figure 7). When comparing the first 4 weeks of the monitoring period for a random patient, the old method (as defined in Example 4) indicated that everything was OK (AMI =100) because no tests were omitted or with results above the BAC-threshold, while as the composite [AMI+MTBET] shows a systematically decreased level ([AMI+MTBET] ~80) due to extreme window gliding giving a median MTBET of 20 h.

This also opens up a method to determine the quality of test schedules. By calculating the maximum time interval a test schedule will permit, including the distance between time points and window size, (without missing tests) gives a quality label for the schedule. Preferred theoretical maximum time between exposure tests is approximately 8 hours. Schedules that results in a theoretical maximum time between exposure tests exceeding 12 hours will make it possible for the patient to conceal drinking should he or she desire to.

Example 6, Maximum Time Between Tests: A Digital Biomarker to Detect Therapy Compliance and Assess Schedule Quality in Measurement-Based eHealth Systems for Alcohol Use Disorder

In a breathalyzer-based eHealth system, the digital biomarker 'maximum time between tests' (MTBT) brings valuable information on alcohol consumption patterns as confirmed by correlation with phosphatidyl ethanol, carbohydrate deficient transferrin and timeline follow-back data. The digital biomarker enables early identification of lapse by patients undergoing therapy for alcohol use disorder by predicting alcohol consumption and misuse of flexible test schedules.

The monitoring of alcohol use has been based mainly on self-reporting using timeline follow-back questionnaires. The introduction of biomarkers (Ref. 1) has provided more objective data on alcohol consumption. In Swedish healthcare, the gold standard is phosphatidyl ethanol (PEth). The major drawback of current disease state-monitoring methods includes poor time resolution and problems with the quality of self-reported data (Ref. 8).

Methods based on self-reporting using smartphone and internet-connected systems have opened up new paths to personalized and continuous care for AUD patients (Ref. 2, Ref. 11). These electronic methods address the recall problem and improve time resolution of self-reporting (e.g. daily, weekly), but reports on clinical efficacy are scarce (Ref. 4, Ref. 9). The latest sensor-based techniques enable objective measurement-based care using either remote frequent (Ref. 3, Ref. 6) or continuous (Ref. 7) real-time monitoring of sobriety.

Digital biomarkers (DBs) are increasingly used to monitor the state of different diseases (Ref. 10). We define a digital biomarker as patient-generated physiological and behavioral measures collected through sensors and other connected digital tools that can be used to monitor, predict and/or influence health-related outcomes.

The eHealth system used in this study has a cloud-connected breathalyzer where the data from measurements are sent to a central database and recalculated, together with test metadata, into different DBs. The first published DB for AUD was the addiction monitoring index which combines the breath alcohol content test results with the pattern of omitted tests (Ref. 6). During clinical trials there were indications that suboptimal test schedules led to secret drinking. Therefore, we hypothesized that time-based parameters extracted from the database might be useful for estimating therapy compliance. One particular metric - maximum time between tests (MTBT) - could be redesigned as a digital biomarker that relies on how a task is conducted rather than what the task produces as a result. In this example we describe the development and verification of MTBT.

Data collection: Previct Alcohol® (Kontigo Care AB) is an eHealth system consisting of a fuel-cell-based pocket-sized breathalyzer connected to a smartphone with an app for the patient and a web-based portal for the care provider (Ref. 5). Data were collected from patients in two clinical trials (Ref. 6). The Previct Alcohol eHealth system was evaluated as an add-on for enhancement of conventional diagnosis, care and aftercare of alcohol-dependent patients. The patients were from both Uppsala University Hospital (n=33) and aftercare patients from three geographical sites of Nämndemansgården (n=21), a private caregiver with a therapy based on the Minnesota model. The number of males was 34 (age 38-73, mean 54.1, SD 8.7) and that of females 20 (age 37-62, mean 50.2, SD 7.0). As we do not compare study groups with controls in this article, we do not discuss the effect of treatment. The time of participation in the study ranged between 3 and 456 days (mean 259, median 314, SD 128).

Timeline follow-back (TLFB) data were collected for a 4-week period at month 1, 2, 3, 6 and 12. For each week the number of standard drinks, drinking days, drinks/drinking day and heavy drinking days ($\geq$4 drinks/day for females; $\geq$5 drinks/day for males) were collected. The total number of patient weeks with TLFB data was 650.

**Scheduling:** The default number of tests in the protocol was 3, but the care providers adapted this to be between 2-4 to accommodate individual needs. The care provider could also select the earliest time point for the first test of the day and the latest possible time point for the last test each day, and a time window for performing the test. The system distributed the tests automatically over the day and always provided at least 1 h between tests. This means that a schedule with 3 tests/day, with time points 7:00 and 22:00 and a window of 2 h yielded a schedule of 7:00-9:00, 13:00-15:00 and 20:00-22:00. The total number of scheduled tests was 2/day for 14 patients, 3/day for 28 patients, between 2 and 3/day for 9 patients, between 3 and 4/day for 1 patient and 2, 3 or 4/day for 2 patients. The number of scheduled tests was 37359, of which 23521 (63%) were performed.

**Biomarkers:** Phosphatidyl ethanol (PEth, 16:0/18:1, in $\mu$mol/L) in blood was measured by LC-MS/MS. PEth measurements were available from 40 patients, 1-6 each (total n = 148). Values below the detection limit (0.05 $\mu$mol/L) were transcripted to 0.025. Carbohydrate deficient transferrin (CDT) was measured by HPLC.

**Maximum Time Between Tests (MTBT):** The time between two consecutive tests (TBT) was calculated and stored in the database as whole hours. Maximum Time Between Tests (MTBT) is the longest TBT on each day. Logarithmically (24 log) transformed weekly averages of MTBT data was used in the correlation studies.

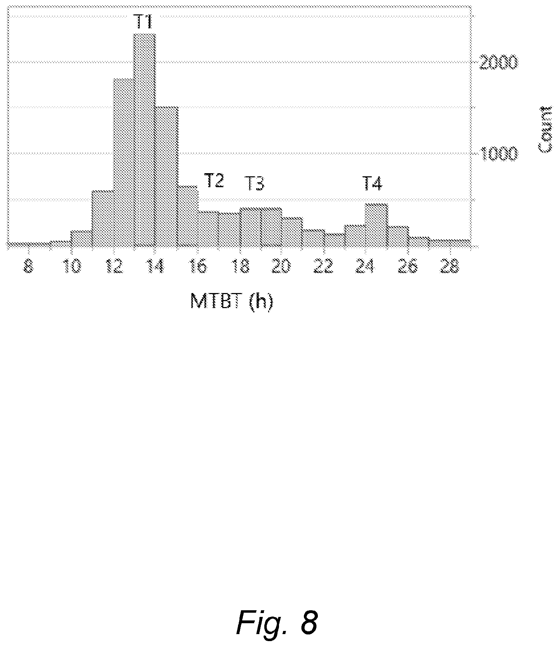

Figure 8 shows the distribution of MTBT data in the 8-28 h range. The existence of data at longer times (~>14h) are due to poor test compliance. Higher MTBT data is excluded for clarity reasons,

**Results:** The test schedules used by the caregivers resulted in an MTBT of 10-14 h and a median MTBT of 13 h (See Figure 8). The factors influencing MTBT are the time point for the first and last test per day, and compliance in the form of ignored tests and sliding within the allowed test window. This gave a distribution with several peaks where the peak distributed around 13 h corresponded to good test compliance (T1 in Figure 8). Poor test compliance resulted in the shoulder at around 16 h (T2, morning or evening test omitted, and/or maximal use of time windows), peaks at 18-20 h (T3, one additional test omitted) and around 24 h (T4, only one test per day performed).

To verify whether increased MTBT could be connected with alcohol consumption we analyzed the correlation between MTBT and data from self-reporting and biomarkers. The model between weekly averages of 24-log transformed MTBT and timeline follow-back data (n = 650) was highly significant (P < 0.0001, R= 0.27-0.38) for: i) number of standard drinks/week, ii) number of drinking days/week, iii) number of drinks/drinking day, and iv) number of heavy drinking days/week. Significant correlations between MTBT and PEth (P < 0.0001, R= 0.41, n = 148) and between MTBT and CDT (P < 0.0079, R = 0.22, n = 120) were noted.

Discussion: We introduced a time-based digital biomarker Maximum Time Between tests (MTBT) for evaluating test schedule quality, test compliance and early detection of lapse/relapse. The scheduling of eHealth systems includes: 1) selecting the number of tests, 2) positioning of tests and 3) defining the size of window allowed for performing each test. The test compliance includes 4) misuse of the time window (shifting) and 5) omission of tests. Poor scheduling parameters and poor test compliance can be misused by the patient for secret drinking and can thus lead to a decreased effect of the system. The significant correlation between MTBT and self-reported alcohol consumption, and between MTBT and the biomarkers PEth and CDT verify that increased MTBT is at least partly related to increased alcohol usage. Therefore, continuous statistical monitoring of MTBT data provides a stable foundation for identification of changes in patient behavior patterns, which in turn can be early signs of lapse.

Sliding within the allowed test window increases the likelihood of a slip, without seemingly influencing test performance statistics and thus avoiding the caregiver being alerted. Already a maximal misuse of a 2 h window for two consecutive tests (evening test early/morning test late in the test window) gives an extra 4 h sobering time. This was initially detected as an extra peak at 14-17 h in the distribution of MTBT data (Fig 1, T2). Since humans metabolize about 0.1-0.2 per mille alcohol per hour, four hours correspond to the body combustion of 2-4 standard drinks, depending on body weight. MTBT is an efficient digital biomarker for the identification of poor 'micro' test compliance caused by sliding within test windows.

Omitted tests are automatically reported to the caregiver through the eHealth system. The omission of the first or last test of the day opens up the possibility of consuming an additional 3-4 drinks and still being able to perform a negative BrAC-test. These omissions were often combined with sliding within the window, increasing the typical MTBT of 10-14h to 16-21 h (3-4 tests/day schedule) and 22-26 h (2 tests/day schedule). Our data suggest that frequently omitted evening and/or morning tests are strong indications of ongoing secret drinking. If MTBT increases and treatment aims for abstinence, then selective biomarker-based assays (e.g. PEth and ethyl glucuronide) should be combined with the eHealth system.

The quality of a test schedule can be evaluated with MTBT. That is, MTBT is a metric of the longest time window for potential undetected secret drinking. The ideal MTBT is in most cases not dependent on the number of scheduled tests/day (if $\geq 2$ and MTBT $\geq 12$ h), i.e. the longest time between two tests is during the night rest. An ideal schedule plan with 3-4 tests/day that keeps MTBT short is characterized by a morning test directly after waking up (early) and an evening test directly before the night rest. It is possible to create a 3 tests/day schedule giving an MTBT in the 8-9 h range and thus minimizing the window for undetected lapses. Schedules with only 2 tests/day might still be an alternative due to personal integrity or practical reasons, when both tests are performed. Four and more tests/day may be used for patients who do not have a daily work/school activity. A quality scheduling has the shortest possible MTBT, preferably including at least three tests, and has small test windows (1-2 h max), all while still being practical for the patient.

In order for a measurement-based scheduled eHealth system to provide sufficient daily support to refrain from the use of alcohol, the scheduling of tests is critical. A flexible system allows the therapist and patient to agree on a convenient schedule compatible with the patient's everyday life; this increases the patient's willingness to use the eHealth solution and to comply with the test schedule, compared to monitoring methods that interfere with daily life and/or patient integrity. However, the flexibility has to be used with care. The 'dosing' of an eHealth system consists of test scheduling and detailed statistical monitoring of test behavior using time-based digital biomarkers. The time-based digital biomarker described here has the potential to become a generally useful metric for all scheduled measurement-based eHealth systems to monitor test behavior and compliance.

**References for Example 6:**

1. Andresen-Streichert H, Müller A, Glah A et al. (2018) Alcohol Biomarkers in Clinical and Forensic Contexts. Dtsch Arztebl Int. 115(18):309-315.

2. Beckjord E, Shiffman S. (2014) Background for real-time monitoring and intervention related to alcohol use. Alcohol Res 36:9-18.

3. Gordon A, Jaffe A, McLellan AT et al. (2017) How should remote clinical monitoring be used to treat alcohol use disorders? Initial findings from an expert round table discussion. J Addict Med 11:145-153.

4. Gustafson DH, McTavish FM, Chih MY et al. (2014) A smartphone application to support recovery from alcoholism: A randomized clinical trial. JAMA Psychiatry 71:566-72.

5. Hämäläinen MD, Andersson K. (2016) Method and device for estimating a risk of relapse of addictive behaviour. WO2016178617.

6. Hämäläinen MD, Zetterström A, Winkvist M et al. (2018) Real-time monitoring using a breathalyzer-based eHealth system can identify lapse/relapse patterns in alcohol use disorder patients. Alcohol Alcohol 54:368-375.

7. Leffingwell TR, Cooney NJ, Murphy JG et al. (2013) Continuous objective monitoring of alcohol use: twenty-first century measurement using transdermal sensors. Alcohol Clin Exp Res 37:16-22.

8. Midanik LT. (1988) Validity of self-reported alcohol use: a literature review and assessment. Br J Addict. 83:1019-29.

9. Rose GL, Badger GJ, Skelly J M et al. (2017) Randomized Controlled Trial of Brief Intervention by Interactive Voice Response, Alcohol Alcohol 52:335-343.

10. Snyder CW, Dorsey ER, Atreja A. (2018) The best digital biomarker papers of 2017. Digit Biomark 2:64-75.

11. Quanbeck A, Chih M-Y, Isham A et al. (2014) Mobile Delivery of Treatment for Alcohol Use Disorders: A Review of the Literature. Alcohol Res 36:111-122.

**Claims**

1. Method for estimating a risk of relapse of addictive behavior of an individual, said addictive behavior being related to exposure to alcohol, said method comprising:

   • providing (S10) a predetermined schedule for said individual to conduct exposure tests, said exposure test comprising performing a determination of said individual's exposure to alcohol, said predetermined schedule comprising multiple points in time with time-slots within which said exposure tests should be conducted;
   • requesting (S20) said individual to conduct exposure tests according to said predetermined schedule;
   • collecting (S30), with a measurements device (303), data from conducted exposure tests, said collected data comprising measurements of said individual's exposure to alcohol and time-points when said exposure tests were conducted;
   • transmitting (S40) said collected data from said measurement device (303) to a central server unit (301);
   • storing (S50) said collected data in said central server unit (301);
   • evaluating (S60) a behavioral pattern related to said individual's compliance to said scheduled exposure tests, based on a determination if said exposure tests are systematically shifted within acceptable time-slots so as to widen the unmonitored time for consuming alcohol in a physically undetectable manner;
   wherein said behavioral pattern is evaluated based on a Maximum Time Between Exposure Tests, MTBET, where MTBET is the maximum time of all "time between exposure tests" that have been collected during a specific period of time, where "time between exposure tests" is the time between two adjacent exposure tests that both are conducted according to said predetermined schedule;
   • estimating (S70) a risk of relapse based on said evaluated behavioral pattern and said measurements of said individual's exposure to alcohol, wherein an elevated MTBET is interpreted as elevated risk for relapse of addictive behavior; and
   • if said estimated risk is greater than a predefined level, transmitting (S80) a message to a third party (304).

2. Method of claim 1, wherein said evaluation of behavioral pattern of how said exposure tests were conducted is further based on evaluating quality parameters of said determination for determining if said individual is trying to tamper with said determination.

3. Method of any preceding claim, wherein said exposure test further comprises answering a questionnaire provided in said measurement device, and said collected data from said conducted exposure tests further comprises said individual's questionnaire answers; and wherein said risk of relapse is estimated further based on said individual's questionnaire answers.

4. Method of any preceding claim, further comprising determining a new point in time, in addition to the already scheduled exposure tests, for requesting said individual to conduct an exposure test, based on said estimated risk for relapse.

5. Method of any preceding claim, further comprising, if said estimated risk is greater than a predefined level, at least one of

   transmitting (S90) a message to said individual;
   activating (S100) an actuator in said measurement device (303).

6. Method of any preceding claim, further comprising identifying said individual during measurement of said individual's exposure to alcohol.

7. Method of any preceding claim, wherein said estimating a risk of relapse is further based on said individual's participation in a support program or treatment program related to addictive behavior.

8. Method of any preceding claim, wherein said estimating a risk of relapse is further based on at least one of the following:

   time of day when measuring said individual's exposure to alcohol;
   day of week when measuring said individual's exposure to alcohol;
   type of day when measuring said individual's exposure to alcohol;
   result of previous measurement of said individual's exposure to alcohol;

number of historic measurements of said individual's exposure to alcohol; that have been missed.

9. Server unit (301) configured to estimate a risk of relapse of addictive behavior of an individual, said addictive behavior being related to exposure to alcohol, wherein the server unit (301) is configured to:

provide a predetermined schedule for said individual to conduct exposure tests, said exposure test comprising performing a measurement of said individual's exposure to alcohol, said predetermined schedule comprising multiple points in time with time-slots within which said exposure tests should be conducted;
request said individual to conduct exposure tests according to said predetermined schedule;
receive, from a measurement device (303) data collected from said exposure tests, said collected data comprising determination of said individual's exposure to alcohol and time-points when said exposure tests were conducted;
store said received data;
evaluate a behavioral pattern related to said individual's compliance to said scheduled exposure tests, based on a determination if said exposure tests are systematically shifted within acceptable time-slots so as to widen the unmonitored time for consuming alcohol in a physically undetectable manner;
wherein said behavioral pattern is evaluated based on a Maximum Time Between Exposure Tests, MTBET, where MTBET is the maximum time of all "time between exposure tests" that have been collected during a specific period of time, where "time between exposure tests" is the time between two adjacent exposure tests that both are conducted according to said predetermined schedule;
estimate a risk of relapse based on said evaluated behavioral pattern and said measurements of said individual's exposure to alcohol, wherein an elevated MTBET is interpreted as elevated risk for relapse of addictive behavior; and
if said estimated risk is greater than a predefined level, transmit a message to a third party (304).

10. Server unit (301) of claim 9, wherein the server unit (301) is further configured to, if said estimated risk is greater than a predefined level, at least one of

transmit a message to said individual;
activate an actuator in said measurement device (303).

11. Server unit of claim 9 or 10, wherein the server unit is further configured to identify said individual during measurement of said individual's exposure to alcohol.

12. System configured to estimate a risk of relapse of addictive behavior of an individual, said addictive behavior being related to exposure to alcohol, wherein the system comprises a server unit (301) of any of the claims 9 to 11, and a measurement device (303) configured to enable estimation of a risk of relapse of addictive behavior of an individual, said addictive behavior being related to exposure to alcohol, wherein the measurement device (303) is configured to:
At multiple points in time, according to a predetermined schedule,
perform a measurement of said individual's exposure to alcohol; and transmit data from said exposure to alcohol measurement to a central server unit (301) to enable estimation of a risk of relapse.

13. System of claim 12, wherein said measurement device (303) is further configured to, at multiple points in time according to said predetermined schedule, provide a questionnaire to said individual, collect said individual's questionnaire answers, and transmit data from said questionnaire answers to the central server unit (301) to enable estimation of a risk of relapse.

14. System of claim 12 or 13, wherein the measurement device (303) comprises at least one of the following:

a breathalyzer configured to measure an alcohol content in said individual's breath;
a motion sensor configured to measure motion of said individual;
a pulse sensor configured to measure pulse of said individual;
a balance configured to measure a weight of said individual.
a smart mobile phone configured to at least one of the following:

present questions, collect answers and communicate with a central server unit (301);
monitor said individual's engagement in online gambling or gaming.

**Patentansprüche**

1. Verfahren zur Schätzung eines Risikos des Rückfalls des Suchtverhaltens einer Person, wobei das Suchtverhalten mit Alkoholkonsum zusammenhängt, wobei das Verfahren Folgendes umfasst:

   • Vorsehen (S10) eines vorab bestimmten Zeitplans zum Durchführen von Alkoholkonsumtests durch die Person, wobei ein Alkoholkonsumtest das Durchführen einer Bestimmung des Alkoholkonsums der Person umfasst, wobei der vorab bestimmte Zeitplan mehrere Zeitpunkte mit Zeitfenstern umfasst, innerhalb derer die Alkoholkonsumtests durchgeführt werden sollten;
   • Auffordern (S20) der Person zum Durchführen von Alkoholkonsumtests gemäß dem vorab bestimmten Zeitplan;
   • Sammeln (S30) von Daten in den durchgeführten Alkoholkonsumtests mit einem Messgerät (303), wobei die gesammelten Daten die Messungen des Alkoholkonsums der Person und die Zeitpunkte umfassen, wann die Alkoholkonsumtests durchgeführt wurden;
   • Übertragen (S40) der gesammelten Daten von dem Messgerät (303) zu einer zentralen Servereinheit (301);
   • Speichern (S50) der gesammelten Daten in der zentralen Servereinheit (301);
   • Auswerten (S60) eines Verhaltensmusters bezüglich der Einhaltung der geplanten Alkoholkonsumtests durch die Person auf Grundlage einer Bestimmung, ob die Alkoholkonsumtests systematisch innerhalb akzeptabler Zeitfenster verschoben werden, um so die nicht überwachte Zeit beim Konsumieren von Alkohol in einer physisch nicht entdeckbaren Weise zu erweitern;
   wobei das Verhaltensmuster auf Grundlage einer maximalen Zeit zwischen den Alkoholkonsumtests ("Maximum Time Between Exposure Tests", MTBET) ausgewertet wird, wobei MTBET die maximale Zeit jeglicher "Zeit zwischen den Alkoholkonsumtests" ist, die während einer bestimmten Zeitperiode gesammelt worden sind, wobei die "Zeit zwischen den Alkoholkonsumtests" die Zeit zwischen zwei aufeinander folgenden Alkoholkonsumtests ist, die beide gemäß dem vorab bestimmten Zeitplan durchgeführt werden;
   • Schätzen (S70) eines Risikos des Rückfalls auf Grundlage des ausgewerteten Verhaltensmusters und der Messungen des Alkoholkonsums der Person, wobei eine erhöhte MTBET als erhöhtes Risiko für den Rückfall des Suchtverhaltens interpretiert wird; und
   • falls das geschätzte Risiko größer als ein vorab definierter Wert ist, Übertragen (S80) einer Meldung an eine dritte Partei (304).

2. Verfahren nach Anspruch 1, wobei die Auswertung des Verhaltensmusters, wie die Alkoholkonsumtests durchgeführt wurden, weiterhin auf Grundlage der Auswertung von Qualitätsparametern der Bestimmung erfolgt, um zu bestimmen, ob die Person versucht, die Bestimmung zu verfälschen.

3. Verfahren nach einem vorstehenden Anspruch, wobei der Alkoholkonsumtest weiterhin das Beantworten eines in dem Messgerät vorhandenen Fragebogens umfasst, und die gesammelten Daten in den durchgeführten Alkoholkonsumtests weiterhin die Antworten der Person auf den Fragebogen enthalten; und wobei das Risiko des Rückfalls weiterhin auf Grundlage der Antworten der Person auf den Fragebogen geschätzt wird.

4. Verfahren nach einem vorstehenden Anspruch, das weiterhin das Bestimmen eines neuen Zeitpunkts, zusätzlich zu den bereits geplanten Alkoholkonsumtests, zum Auffordern der Person zum Durchführen eines Alkoholkonsumtests auf Grundlage des geschätzten Risikos des Rückfalls umfasst.

5. Verfahren nach einem vorstehenden Anspruch, das weiterhin umfasst, ob das geschätzte Risiko größer als ein vorab definierter Wert ist, mindestens eines von

   Übertragen (S90) einer Meldung an die Person;
   Aktivieren (S100) eines Aktuators in dem Messgerät (303).

6. Verfahren nach einem vorstehenden Anspruch, das weiterhin das Identifizieren der Person während der Messung des Alkoholkonsums der Person umfasst.

7. Verfahren nach einem vorstehenden Anspruch, wobei die Schätzung eines Risikos des Rückfalls weiterhin auf Grundlage der Teilnahme der Person an einem Supportprogramm oder Behandlungsprogramm bezüglich des Suchtverhaltens erfolgt.

8. Verfahren nach einem vorstehenden Anspruch, wobei die Schätzung eines Risikos des Rückfalls weiterhin auf Grundlage mindestens eines der Folgenden erfolgt:

> Tageszeit, wann der Alkoholkonsum der Person gemessen wird;
> Wochentag, wann der Alkoholkonsum der Person gemessen wird;
> Art des Tages, wann der Alkoholkonsum der Person gemessen wird;
> Ergebnis einer vorherigen Messung des Alkoholkonsums der Person;
> Anzahl der historischen Messungen des Alkoholkonsums der Person; die verpasst wurden.

9. Servereinheit (301), die ausgebildet ist, ein Risiko des Rückfalls des Suchtverhaltens einer Person zu schätzen, wobei das Suchtverhalten mit Alkoholkonsum zusammenhängt, wobei die Servereinheit (301) ausgebildet ist:

> einen vorab bestimmten Zeitplan zum Durchführen von Alkoholkonsumtests durch die Person vorzusehen, wobei der Alkoholkonsumtest das Durchführen einer Messung des Alkoholkonsums der Person umfasst, wobei der vorab bestimmte Zeitplan mehrere Zeitpunkte mit Zeitfenstern umfasst, innerhalb derer die Alkoholkonsumtests durchgeführt werden sollten;
> die Person zum Durchführen von Alkoholkonsumtests gemäß dem vorab bestimmten Zeitplan aufzufordern;
> von einem Messgerät (303) in den Alkoholkonsumtests gesammelte Daten zu empfangen, wobei die gesammelten Daten die Bestimmung des Alkoholkonsums der Person und die Zeitpunkte umfassen, wann die Alkoholkonsumtests durchgeführt wurden;
> die empfangenen Daten zu speichern;
> ein Verhaltensmuster bezüglich der Einhaltung der geplanten Alkoholkonsumtests durch die Person auf Grundlage einer Bestimmung auszuwerten, ob die Alkoholkonsumtests systematisch innerhalb akzeptabler Zeitfenster verschoben werden, um so die nicht überwachte Zeit beim Konsumieren von Alkohol in einer physisch nicht entdeckbaren Weise zu erweitern;
> wobei das Verhaltensmuster auf Grundlage einer maximalen Zeit zwischen den Alkoholkonsumtests ("Maximum Time Between Exposure Tests", MTBET) ausgewertet wird, wobei MTBET die maximale Zeit jeglicher "Zeit zwischen den Alkoholkonsumtests" ist, die während einer bestimmten Zeitperiode gesammelt worden sind, wobei die "Zeit zwischen den Alkoholkonsumtests" die Zeit zwischen zwei aufeinander folgenden Alkoholkonsumtests ist, die beide gemäß dem vorab bestimmten Zeitplan durchgeführt werden;
> ein Risiko des Rückfalls auf Grundlage des ausgewerteten Verhaltensmusters und der Messungen des Alkoholkonsums der Person zu schätzen, wobei eine erhöhte MTBET als erhöhtes Risiko des Rückfalls des Suchtverhaltens interpretiert wird; und
> falls das geschätzte Risiko größer als ein vorab definierter Wert ist, eine Meldung an eine dritte Partei (304) zu übertragen.

10. Servereinheit (301) nach Anspruch 9, wobei die Servereinheit (301) weiterhin, falls das geschätzte Risiko größer als ein vorab definierter Wert ist, zu mindestens einem von Folgenden ausgebildet ist:

> Übertragen einer Meldung an die Person;
> Aktivieren eines Aktuators in dem Messgerät (303).

11. Servereinheit nach Anspruch 9 oder 10, wobei die Servereinheit ausgebildet ist, die Person während der Messung des Alkoholkonsums der Person zu identifizieren.

12. System, das ausgebildet ist, ein Risiko des Rückfalls des Suchtverhaltens einer Person zu schätzen, wobei das Suchtverhalten mit Alkoholkonsum zusammenhängt, wobei das System eine Servereinheit (301) nach einem der Ansprüche 9 bis 11 und ein Messgerät (303) umfasst, das ausgebildet ist, eine Schätzung eines Risikos des Rückfalls des Suchtverhaltens einer Person zu ermöglichen, wobei das Suchtverhalten mit Alkoholkonsum zusammenhängt, wobei das Messgerät (303) ausgebildet ist:

> an mehreren Zeitpunkten gemäß einem vorab bestimmten Zeitplan eine Messung des Alkoholkonsums der Person durchzuführen; und
> Daten aus der Messung des Alkoholkonsums an eine zentrale Servereinheit (301) zu übertragen, um eine Schätzung eines Risikos des Rückfalls zu ermöglichen.

13. System nach Anspruch 12, wobei das Messgerät (303) weiterhin ausgebildet ist, an mehreren Zeitpunkten gemäß dem vorab bestimmten Zeitplan einen Fragebogen für die Person vorzusehen, Antworten der Person auf den Fragebogen zu sammeln, und die Daten aus den Antworten auf den Fragebogen an die zentrale Servereinheit (301) zu übertragen, um eine Schätzung eines Risikos des Rückfalls zu ermöglichen.

14. System nach Anspruch 12 oder 13, wobei das Messgerät (303) mindestens eines der folgenden aufweist:

einen Atemanalysator, der ausgebildet ist, einen Alkoholanteil in dem Atem der Person zu messen;
einen Bewegungssensor, der ausgebildet ist, eine Bewegung der Person zu messen;
einen Pulssensor, der ausgebildet ist, den Puls der Person zu messen;
eine Waage, die ausgebildet ist, das Gewicht der Person zu messen;
ein Smartphone, das zu mindestens einem der folgenden ausgebildet ist:

Fragen zu stellen, Antworten zu sammeln und mit einer zentralen Servereinheit (301) zu kommunizieren;
die Teilnahme der Person an Online-Spielen oder -Glücksspielen zu überwachen.

**Revendications**

1. Procédé d'estimation du risque de rechute d'un comportement addictif d'un individu, ledit comportement addictif étant lié à l'exposition à l'alcool, ledit procédé comprenant :

• la fourniture (S10) d'un calendrier prédéterminé pour que l'individu effectue des tests d'exposition, ledit test d'exposition consistant à déterminer de ladite exposition d'un individu à l'alcool, ledit calendrier prédéterminé comprenant plusieurs points dans le temps avec des créneaux horaires au cours desquels lesdits tests d'exposition doivent être effectués ;
• la demande (S20) audit individu d'effectuer des tests d'exposition selon ledit calendrier prédéterminé ;
• le recueil (S30), à l'aide d'un dispositif de mesures (303), de données provenant des tests d'exposition effectués, lesdites données recueillies comprenant des mesures de ladite exposition de l'individu à l'alcool et des points dans le temps pendant lesquels lesdits tests d'exposition ont été effectués ;
• la transmission (S40) desdites données recueillies par ledit dispositif de mesure (303) à un serveur central (301) ;
• le stockage (S50) desdites données recueillies dans ladite unité de serveur central (301) ;
• l'évaluation (S60) d'un modèle de comportement lié à ladite conformité de l'individu auxdits tests d'exposition programmés, sur la base d'une détermination si lesdits tests d'exposition sont systématiquement décalés dans des créneaux horaires acceptables de manière à élargir la période non surveillée pour la consommation d'alcool d'une manière physiquement indétectable ;
dans lequel ledit modèle de comportement est évalué sur la base d'une durée maximale entre les tests d'exposition, MTBET, où MTBET représente la durée maximale de toutes les « durées entre les tests d'exposition » qui ont été recueillies au cours d'une période de temps spécifique, où « la durée entre les tests d'exposition » représente la durée entre deux tests d'exposition adjacents qui sont tous les deux effectués selon ledit calendrier prédéterminé ;
• l'estimation (S70) d'un risque de rechute sur la base dudit modèle de comportement évalué et desdites mesures de ladite exposition de l'individu à l'alcool, dans lequel une MTBET élevée est interprétée comme un risque élevé de rechute d'un comportement de dépendance ; et
• si ledit risque estimé est supérieur à un niveau prédéfini, la transmission (S80) d'un message à un tiers (304).

2. Procédé selon la revendication 1, dans lequel ladite évaluation du modèle de comportement de la manière dont lesdits tests d'exposition ont été menés est en outre basée sur l'évaluation des paramètres de qualité de ladite détermination afin de déterminer si ledit individu tente de falsifier ladite détermination.

3. Procédé selon l'une quelconque des revendications précédentes, dans lequel ledit test d'exposition comprend en outre la réponse à un questionnaire fourni dans ledit dispositif de mesure, et lesdites données recueillies à partir desdits tests d'exposition effectués comprennent en outre lesdites réponses au questionnaire de l'individu ; et dans lequel ledit risque de rechute est estimé en outre sur la base desdites réponses au questionnaire de l'individu.

4. Procédé selon l'une quelconque des revendications précédentes, comprenant en outre la détermination d'un nouveau point dans le temps, en plus des tests d'exposition déjà programmés, pour demander audit individu d'effectuer un test d'exposition, sur la base de l'estimation du risque de rechute.

5. Procédé selon l'une quelconque des revendications précédentes, comprenant en outre, si ledit risque estimé est supérieur à un niveau prédéfini, au moins l'une des actions suivantes :

transmission (S90) d'un message à l'individu ;
activation (S100) d'un actionneur dans le dispositif de mesure (303).

6. Procédé selon l'une quelconque des revendications précédentes, comprenant en outre l'identification dudit individu lors de la mesure de ladite exposition de l'individu à l'alcool.

7. Procédé selon l'une quelconque des revendications précédentes, dans lequel ladite estimation d'un risque de rechute est en outre basée sur ladite participation de l'individu à un programme de soutien ou à un programme de traitement lié à un comportement addictif.

8. Procédé selon l'une quelconque des revendications précédentes, dans lequel ladite estimation d'un risque de rechute est en outre basée sur au moins l'un des éléments suivants :

l'heure de la journée lors de la mesure de ladite exposition de l'individu à l'alcool ;
le jour de la semaine lors de la mesure de ladite exposition de l'individu à l'alcool ;
le type de jour lors de la mesure de ladite exposition de l'individu à l'alcool ;
le résultat de la mesure précédente de ladite exposition de l'individu à l'alcool ;
le nombre de mesures historiques de ladite exposition de l'individu à l'alcool ; qui ont été omis.

9. Unité de serveur (301) configurée pour évaluer le risque de rechute d'un comportement addictif d'un individu, ledit comportement addictif étant lié à l'exposition à l'alcool, dans laquelle l'unité de serveur (301) est configurée pour :

fournir un calendrier prédéterminé pour que ledit individu effectue des tests d'exposition, ledit test d'exposition comprenant la réalisation d'une mesure de ladite exposition de l'individu à l'alcool, ledit calendrier prédéterminé comprenant de multiples points dans le temps avec des créneaux horaires dans lesquels lesdits tests d'exposition doivent être effectués ;
demander audit individu d'effectuer des tests d'exposition selon ledit programme prédéterminé ;
recevoir, à partir d'un dispositif de mesure (303), des données recueillies à partir desdits tests d'exposition, lesdites données recueillies comprenant la détermination de ladite exposition de l'individu à l'alcool et les points dans le temps pendant lesquels lesdits tests d'exposition ont été effectués ;
stocker lesdites données reçues ;
évaluer un modèle de comportement lié à ladite conformité de l'individu auxdits tests d'exposition programmés, sur la base d'une détermination si lesdits tests d'exposition sont systématiquement décalés dans des créneaux horaires acceptables de manière à élargir la période non surveillée pour la consommation d'alcool d'une manière physiquement indétectable ;
dans laquelle ledit modèle de comportement est évalué sur la base d'une durée maximale entre les tests d'exposition (MTBET), où la MTBET représente la durée maximale de toutes les « durées entre les tests d'exposition » qui ont été recueillies au cours d'une période spécifique, où la « durée entre les tests d'exposition » représente la durée entre deux tests d'exposition adjacents qui sont tous deux effectués conformément audit calendrier prédéfini ;
estimer un risque de rechute sur la base dudit modèle de comportement évalué et desdites mesures de ladite exposition de l'individu à l'alcool,
dans laquelle une MTBET élevée est interprétée comme un risque élevé de rechute du comportement addictif ; et
si ledit risque estimé est supérieur à un niveau prédéfini, transmettre un message à une tierce partie (304).

10. Unité de serveur (301) selon la revendication 9, dans laquelle l'unité de serveur (301) est en outre configurée pour, si ledit risque estimé est supérieur à un niveau prédéfini, au moins l'un des éléments suivants

transmettre un message audit individu ;
activer un actionneur dans ledit dispositif de mesure (303).

11. Unité de serveur selon la revendication 9 ou la revendication 10, dans laquelle l'unité de serveur est en outre configurée pour identifier ledit individu pendant la mesure de ladite exposition de l'individu à l'alcool.

12. Système configuré pour estimer un risque de rechute d'un comportement addictif d'un individu, ledit comportement addictif étant lié à l'exposition à l'alcool, dans lequel le système comprend une unité serveur (301) selon l'une quelconque des revendications 9 à 11, et un dispositif de mesure (303) configuré pour permettre l'estimation d'un risque de rechute d'un comportement addictif d'un individu, ledit comportement addictif étant lié à l'exposition à l'alcool, dans lequel le dispositif de mesure (303) est configuré pour :

à plusieurs points dans le temps, selon un calendrier prédéterminé, effectuer une mesure de ladite exposition de l'individu à l'alcool ; et
transmettre les données provenant de la mesure de l'exposition à l'alcool à une unité centrale de serveur (301) pour permettre l'estimation d'un risque de rechute.

13. Système selon la revendication 12, dans lequel ledit dispositif de mesure (303) est en outre configuré pour, à plusieurs points dans le temps selon ledit calendrier prédéterminé, fournir un questionnaire audit individu, recueillir lesdites réponses au questionnaire de l'individu, et transmettre les données provenant desdites réponses au questionnaire à l'unité centrale de serveur (301) pour permettre l'estimation d'un risque de rechute.

14. Système selon la revendication 12 ou la revendication 13, dans lequel le dispositif de mesure (303) comprend au moins l'un des éléments suivants :

un éthylomètre configuré pour mesurer un taux d'alcool dans ladite haleine de l'individu ;
un capteur de mouvement configuré pour mesurer le mouvement dudit individu ;
un capteur de pouls configuré pour mesurer le pouls dudit individu ;
une balance configurée pour mesurer un poids dudit individu ;
un téléphone mobile intelligent configuré pour au moins l'un des éléments suivants :

présenter des questions, recueillir des réponses et communiquer avec une unité centrale de serveur (301) ;
surveiller ledit engagement de l'individu dans des jeux d'argent ou de hasard en ligne.

START

| PROVIDE SCHEDULE FOR CONDUCTING EXPOSURE TESTS | S10 |

| REQUEST INDIVIDUAL TO CONDUCT EXPOSURE TESTS ACCORDING TO SCHEDULE | S20 |

| COLLECT DATA FROM EXPOSURE TESTS | S30 |

| TRANSMIT COLLECTED DATA TO CENTRAL SERVER | S40 |

| STORE DATA IN CENTRAL SERVER | S50 |

| EVALUATE BEHAVIORAL PATTERN BASED ON TIME-POINTS WHEN EXPOSURE TESTS WERE CONDUCTED | S60 |

| ESTIMATE RISK OF RELAPSE BASED ON BEHAVIORAL PATTERN AND MEASUREMENTS OF EXPOSURE | S70 |

| TRANSMIT MESSAGE TO THIRD PARTY IF RISK IS GREATER THAN A PREDEFINED LEVEL | S80 |

END

## Fig. 1a

S80

TRANSMIT MESSAGE TO INDIVIDUAL IF RISK IS GREATER THAN A PREDEFINED LEVEL — S90

ACTIVATE ACTUATOR IF RISK IS GREATER THAN A PREDEFINED LEVEL — S100

END

*Fig. 1b*

*Fig. 2*

Fig. 3

*Fig. 4*

*Fig. 5*

*Fig. 6*

*Fig. 7*

*Fig. 8*

# EP 3 721 435 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2016178617 A
- US 20170181696 A1
- US 20160196389 A1

### Non-patent literature cited in the description

- **HODGINS, DAVID C ; EL-GUEBALY, NADY**. Retrospective and Prospective Reports of Precipitants to Relapse in Pathological Gambling. *Journal of Consulting and Clinical Psychology*, February 2004, vol. 72 (1), 72-80, http://dx.doi.org/10.1037/0022-006X.72.1.72
- **CHIH**. Predictive Modeling of Addiction Lapses in a Mobile Health Application. *J Subst Abuse Treat.*, January 2014, vol. 46 (1), 29-35
- **LEUNG**. Effect of self-administered auricular acupressure on smoking cessation--a pilot study. *BMC Complement Altern Med*, 28 February 2012
- **RH MOOS ; BS MOOS**. Rates and predictors of relapse after natural and treated remission from alcohol use disorders. *Addiction*, February 2006, vol. 101 (2), 212-222
- **HELANDER**. Monitoring of the alcohol biomarkers PEth, CDT and EtG/EtS in an outpatient treatment setting. *Alcohol Alcohol.*, September 2012, vol. 47 (5), 552-7
- **ANDRESEN-STREICHERT H ; MÜLLER A ; GLAH A et al.** Alcohol Biomarkers in Clinical and Forensic Contexts. *Dtsch Arztebl Int.*, 2018, vol. 115 (18), 309-315
- **BECKJORD E ; SHIFFMAN S.** Background for real-time monitoring and intervention related to alcohol use.. *Alcohol Res*, 2014, vol. 36, 9-18
- **GORDON A ; JAFFE A ; MCLELLAN AT et al.** How should remote clinical monitoring be used to treat alcohol use disorders? Initial findings from an expert round table discussion.. *J Addict Med*, 2017, vol. 11, 145-153
- **GUSTAFSON DH ; MCTAVISH FM ; CHIH MY et al.** A smartphone application to support recovery from alcoholism: A randomized clinical trial.. *JAMA Psychiatry*, 2014, vol. 71, 566-72
- **HÄMÄLÄINEN MD ; ANDERSSON K**. *Method and device for estimating a risk of relapse of addictive behaviour*, 2016
- **HÄMÄLÄINEN MD ; ZETTERSTRÖM A ; WINKVIST M et al.** Real-time monitoring using a breathalyzer-based eHealth system can identify lapse/relapse patterns in alcohol use disorder patients. *Alcohol Alcohol*, 2018, vol. 54, 368-375
- **LEFFINGWELL TR ; COONEY NJ ; MURPHY JG et al.** Continuous objective monitoring of alcohol use: twenty-first century measurement using transdermal sensors.. *Alcohol Clin Exp Res*, 2013, vol. 37, 16-22
- **MIDANIK LT**. Validity of self-reported alcohol use: a literature review and assessment.. *Br J Addict.*, 1988, vol. 83, 1019-29
- **ROSE GL ; BADGER GJ ; SKELLY J M et al.** Randomized Controlled Trial of Brief Intervention by Interactive Voice Response. *Alcohol Alcohol*, 2017, vol. 52, 335-343
- **SNYDER CW ; DORSEY ER ; ATREJA A.** The best digital biomarker papers of 2017. *Digit Biomark*, 2018, vol. 2, 64-75
- **QUANBECK A ; CHIH M-Y ; ISHAM A et al.** Mobile Delivery of Treatment for Alcohol Use Disorders: A Review of the Literature. *Alcohol Res*, 2014, vol. 36, 111-122